# EUROPEAN PATENT APPLICATION

(11) **EP 4 596 528 A1**
(43) Date of publication of application: **06.08.2025**
(21) Application number: 23872553.5
(22) Date of filing: 28.09.2023
(51) Int. Cl.: C07C 211/54, H10K 50/10, H10K 50/15

(54) **COMPOUND, ORGANIC ELECTROLUMINESCENT ELEMENT, AND ELECTRONIC DEVICE**

(30) Priority: 30.09.2022 JP 2022157228
(71) Applicant: Hodogaya Chemical Co., Ltd., Tokyo, 105-0021 (JP)
(72) Inventor: KASE, Kouki, Tokyo 105-0021 (JP); KO, Sang-Won, Tokyo 105-0021 (JP); LIM, Jae-Geon, Tokyo 105-0021 (JP); HIRAYAMA, Yuta, Tokyo 105-0021 (JP); HAYASHI, Shuichi, Tokyo 105-0021 (JP)
(74) Representative: dompatent von Kreisler Selting Werner - Partnerschaft von Patent- und Rechtsanwälten mbB
(86) International application number: PCT/JP2023/035504
(87) International publication number: WO 2024/071332

(57) **Abstract**

The compound represented by the following general formula has excellent hole transport and electron blocking abilities. A, R₁ and R₂ each represent a monovalent aromatic hydrocarbon group, etc.; B and C each represent a naphthylene group; L₁ to L₃ each represent a single bond, or a divalent aromatic hydrocarbon group, etc.

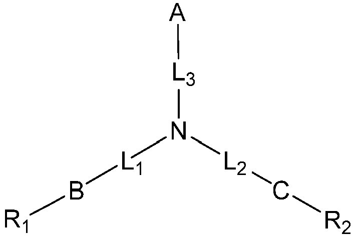

## Description

### Technical Field

The invention relates to a compound useful as electron blocking materials and hole transporting materials, and to an organic electroluminescent device and an electronic device using the compound.

### Background Art

Organic electroluminescent devices (organic EL devices) have been the subject of active research because they are self-luminescent devices, which means they are brighter and more visible than liquid crystal devices, and can provide a clearer display.

In 1987, C.W. Tang et al. of Eastman Kodak Company developed a multilayer structure device in which various roles are assigned to individual materials, as a practical organic EL device. Specifically, they laminate a phosphor that can transport electrons and an organic material that can transport holes, and inject both charges into the phosphor layer to emit light, thus obtaining a high luminance of 1000 cd/m² or more at a voltage of 10 V or less (see, for example, PTL 1 and PTL 2).

To date, many improvements have been made for the practical application of organic EL devices, and high efficiency and durability have been achieved by further subdividing the various roles of the laminate structure into a laminate structure with an anode, a hole injection layer, a hole transporting layer, a light emitting layer, an electron transporting layer, an electron injection layer, and a cathode sequentially arranged on a substrate (see, for example, NPL 1).

In addition, the use of triplet excitons has been attempted to further improve luminous efficiency, and the use of phosphorescent emissive compounds has been investigated (see, for example, NPL 2). In addition, devices utilizing luminescence emitted by thermally activated delayed fluorescence (TADF) have been developed, and in 2011, Adachi et al. of Kyushu University realized an external quantum efficiency of 5.3% with a device using a thermally activated delayed fluorescent material (See, for example, NPL 3).

The light emitting layers of these organic EL devices are made by doping a fluorescent or phosphorescent compound or a material that emits delayed fluorescence into a charge-transporting compound generally called a host material. As described in the above non-patent literature, various organic layers are provided in organic EL devices, and the choice of organic materials has a significant impact on various properties such as the efficiency and durability of the devices (see, for example, NPL 2).

In other words, in an organic EL device, the charges injected from both electrodes are recombined in the light emitting layer to produce luminescence, so it is important how efficiently both charges of holes and electrons are transferred to the light emitting layer, and the device must have an excellent carrier balance. For example, by using a material with hole injectivity that supplies holes injected from the anode to the light emitting layer and a material with electron blocking properties that block electrons injected from the cathode, the probability of recombination between holes and electrons in the light emitting layer can be improved and further, excitons generated in the light emitting layer can be confined to attain high luminous efficiency. Therefore, hole transporting materials must have high hole mobility, high electron blocking power, and high durability against electrons.

Heat resistance and amorphousness of materials are also important with respect to the lifetime of devices. In materials with low heat resistance, the heat generated during device drive causes thermal decomposition even at low temperatures, resulting in material degradation. Materials with low amorphousness cause crystallization of thin films and device degradation even in a short period of time. Consequently, the materials to be used must have high heat resistance and good amorphousness.

Heretofore, N,N'-diphenyl-N,N'-di(α-naphthyl)benzidine (NPD) and various aromatic amine derivatives have been used as hole transporting materials for organic EL devices (see, for example, PTL 1 and PTL 2). However, although NPD has good hole transport ability, its glass transition point (Tg), an index of heat resistance, is low at 96°C, and under high temperature conditions, device characteristics deteriorate due to crystallization (see, for example, NPL 4).

Some aromatic amine derivatives mentioned above can have an excellent hole mobility of 10⁻³ cm²/Vs or higher (see, for example, PTL 1 and PTL 2); however, since their electron blocking power are insufficient, some electrons can pass through the light emitting layer with the derivative and no improvement in luminous efficiency can be expected. Therefore, to achieve even higher efficiency, materials with higher electron blocking power and with ability to form more stable and more heat-resistant thin films are required. There are also reports of aromatic amine derivatives with high durability (see, for example, PTL 3), but in these, the derivatives were used as charge transporting materials for electrophotographic photoreceptors, and there were no examples of their use as organic EL devices.

To solve this problem, substituted carbazole structures and arylamine compounds have been proposed as compounds with improved properties such as heat resistance and hole injection properties (see, for example, PTL 4 and PTL 5), in which, however, improvements have been made in the lifetime and the luminous efficiency in the devices that use these compounds in the hole injection or hole transporting layers, but are still not sufficient, and consequently, a further lower drive voltage, a higher luminous efficiency, and a longer device lifetime are required.

### Citation List

### Patent Literature

PTL 1: US5792557B
PTL 2: US5639914B
PTL 3: US7759030B
PTL 4: JP2009-076817A
PTL 5: JP6674892B
PTL 6: EP2684932B
PTL 7: KR1020200131929A
PTL 8: WO2017/073594A1

### Non Patent Literature

NPL 1: The 9th Symposium of the Society for Applied Physics, Proceedings pp. 55-61 (2001)
NPL 2: The 9th Symposium of the Society for Applied Physics, Proceedings pp. 23-31 (2001)
NPL 3: Appl. Phys. Let., 98, 083302 (2011)
NPL 4: The 3rd Regular Meeting of the Organic EL Discussion Committee, Proceedings pp. 13-14 (2006)

### Summary of Invention

### Technical Problem

As described above, aromatic amine compounds having various functions have been proposed as materials for organic EL devices. However, the fact is that compounds with both excellent hole transport and electron blocking abilities and high thermal stability have not yet been realized.

An object of the invention is to provide a material for organic EL devices with excellent hole transport and electron blocking abilities and high thermal stability in the thin film state. In addition, it is to provide an organic EL device with a low drive voltage, and high luminous efficiency and power efficiency, and a long device lifetime.

### Solution to Problem

The present inventors have promoted assiduous studies for attaining the above-mentioned object and, as a result, have found that an amine compound with at least two naphthylene groups to which an aromatic hydrocarbon group or an aromatic heterocyclic group bonds have excellent hole transport and electron blocking abilities and high thermal stability in the thin film state. With that, the inventors have further found that an organic EL device with a low drive voltage and high luminous efficiency and power efficiency can be realized, using the amine compound. The invention has been proposed based on such findings, and specifically has the following constitution.

1) A compound represented by the following general formula (I): 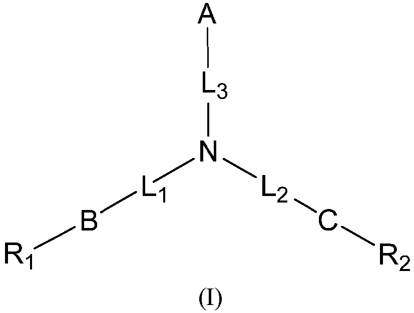 in the formula,
   A represents:
   a substituted or unsubstituted monovalent aromatic hydrocarbon group, or
   a substituted or unsubstituted monovalent aromatic heterocyclic group,
   B and C can be the same as or different from each other,
   each representing a heavy hydrogen-substituted or unsubstituted naphthylene group,
   R₁ and R₂ can be the same as or different from each other,
   each representing a heavy hydrogen-substituted or unsubstituted monovalent aromatic hydrocarbon group, or
   a heavy hydrogen-substituted or unsubstituted monovalent aromatic heterocyclic group,
   L₁, L₂ and L₃ can be the same as or different from each other,
   each representing a single bond,
   a heavy hydrogen-substituted or unsubstituted divalent aromatic hydrocarbon group, or
   a heavy hydrogen-substituted or unsubstituted divalent aromatic heterocyclic group, provided that:
      A-L₃ is not an unsubstituted aromatic heterocyclic group.
2) The invention is also the compound described in the above 1), in which, in the above general formula (I),
   B and C can be the same as or different from each other,
   each representing a heavy hydrogen-substituted or unsubstituted 1,2-naphthylene group,
   a heavy hydrogen-substituted or unsubstituted 1,3-naphthylene group,
   a heavy hydrogen-substituted or unsubstituted 1,4-naphthylene group,
   a heavy hydrogen-substituted or unsubstituted 2,4-naphthylene group,
   a heavy hydrogen-substituted or unsubstituted 2,5-naphthylene group,
   a heavy hydrogen-substituted or unsubstituted 2,6-naphthylene group, or
   a heavy hydrogen-substituted or unsubstituted 2,8-naphthylene group.
3) The invention is also the compound described in the above 1) or 2), in which, in the above general formula (I),
   L₁ and L₂ each represent
   a heavy hydrogen-substituted or unsubstituted phenylene group, or
   a heavy hydrogen-substituted or unsubstituted biphenylylene group.
4) The invention is also the compound described in any of the above 1) to 3), in which, in the above general formula (I),
   R₁ and R₂ can be the same as or different from each other,
   each representing a heavy hydrogen-substituted or unsubstituted phenyl group,
   a heavy hydrogen-substituted or unsubstituted naphthyl group,
   a heavy hydrogen-substituted or unsubstituted dibenzofuranyl group,
   a heavy hydrogen-substituted or unsubstituted phenanthrenyl group, or
   a heavy hydrogen-substituted or unsubstituted biphenyl group.
5) The invention is also the compound described in any of the above 1) to 4), in which, in the above general formula (I),
   A represents:
   a substituted or unsubstituted phenyl group,
   a substituted or unsubstituted naphthyl group,
   a substituted or unsubstituted dibenzofuranyl group,
   a substituted or unsubstituted dibenzothienyl group,
   a substituted or unsubstituted phenanthrenyl group, or
   a substituted or unsubstituted biphenyl group.
6) The invention is also the compound described in any of the above 1) to 5), in which, in the above general formula (I),
   B and C represent the same group.
7) The invention is also the compound described in the above 6), in which, in the above general formula (I),
   R₁ and R₂ represent the same group.
8) The invention is also the compound described in the above 6), in which, in the above general formula (I),
   R₁ and R₂ represent different groups.
9) The invention is also the compound described in any of the above 1) to 5), in which, in the above general formula (I),
   B and C represent different groups.
10) The invention is also the compound described in the above 9), in which, in the above general formula (I),
   R₁ and R₂ represent the same group.
11) The invention is also the compound described in the above 9), in which, in the above general formula (I),
   R₁ and R₂ represent different groups.
12) The compound of any of the above 1) to 11), in which an absolute value of the HOMO level of the compound represented by the above general formula (I), as measured with a thin film of 100 nm thickness prepared by vacuum evaporation of the compound represented by the above general formula (I) on a substrate with indium tin oxide (ITO), using an ionization potential measuring device in vacuum, is 5.60 eV or more and 5.80 eV or less.
13) An electron blocking material including the compound described in any of the above 1) to 12).
14) An organic EL device having a pair of electrodes and organic layers containing at least a light emitting layer arranged between the pair of electrodes, the organic EL device containing the compound described in any of the above 1) to 12), in the at least one organic layer therein.
15) The organic EL device described in the above 14), in which the at least one organic layer is an electron blocking layer and the electron blocking layer contains the compound.
16) The organic EL device described in the above 15), in which the at least one organic layer is a hole transporting layer and the hole transporting layer contains a compound represented by the following general formula (II): 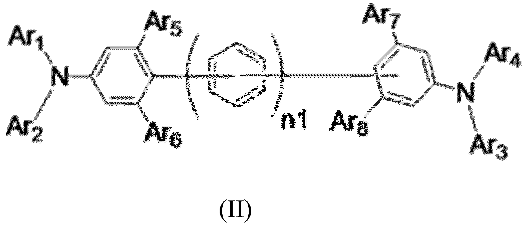
   in the formula, Ar₁ to Ar₅ can be the same as or different from each other, representing a substituted or unsubstituted monovalent aromatic hydrocarbon group;
   Ar₆ to Ar₈ can be the same as or different from each other, representing a hydrogen atom, or a substituted or unsubstituted monovalent aromatic hydrocarbon group, and at least two of Ar₆ to Ar₈ are hydrogen atoms;
   n1 represents 0, 1 or 2;
   Ar₃ and Ar₄ can bond to each other via a single bond to form a ring, or can bond to each other via a substituted or unsubstituted methylene group, an oxygen atom, or a sulfur atom to form a ring;
   Ar₃ or Ar₄ can bond to the benzene ring to which -N(Ar₃)(Ar₄) bonds via a single bond to form a ring, or can bond thereto via a substituted or unsubstituted methylene group, an oxygen atom, or a sulfur atom to form a ring.
17) The organic EL device described in the above 16), in which an absolute value of the HOMO level of the compound represented by the above general formula (II), as measured with a thin film of 100 nm thickness prepared by vacuum evaporation of the compound represented by the above general formula (II) on a substrate with ITO, using an ionization potential measuring device in vacuum, is 5.50 eV or more and 5.65 eV or less.
18) The organic EL device described in the above 16) or 17), in which an absolute value of the HOMO level difference between the compound represented by the above general formula (I) and the compound represented by the above general formula (II) is 0.05 eV or more and 0.35 eV or less.
19) An electronic device having a pair of electrodes and at least one organic layer arranged between the pair of electrodes, in which the at least one organic layer contains the compound described in any of the above 1) to 12).

### Advantageous Effects of Invention

The compound of the invention is excellent in hole transporting and electron blocking abilities and has high thermal stability in the thin film state, and is therefore useful as an electron blocking material and a hole transporting material. An organic EL device using the compound of the invention as a material of the organic layer therein can realize a low drive voltage, a high luminous efficiency, and a long device lifetime.

### Brief Description of Drawings

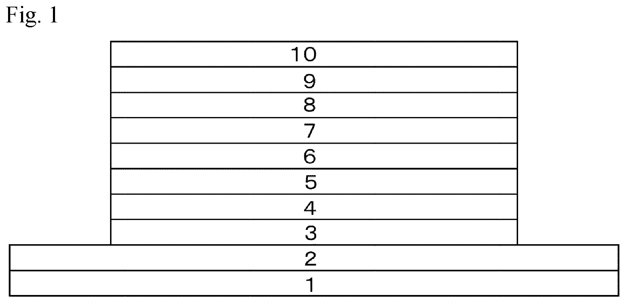

[Fig. 1] Fig. 1 is a schematic cross-sectional view showing a layer configuration of the organic EL devices produced in Examples 23 to 120 and Comparative Examples 1 to 5.

### Description of Embodiments

The contents of the invention will be described in detail below. The constitutional elements can be described below with reference to representative embodiments and specific examples of the invention, but the invention is not limited to the embodiments and the examples. In the present description, a numerical range expressed using "to" means a range that includes the numerical values described before and after "to" as the lower limit and the upper limit. The hydrogen atom that is present in the molecule of the compound used in the invention is not particularly limited in isotope species, and for example, all the hydrogen atoms in the molecule can be ¹H, and all or a part of them can be ²H (deuterium D). In the present description, the term "substituted or unsubstituted" means that the group to which the term is attached can be an unsubstituted group (a group whose hydrogen atoms are not substituted with substituents) or that at least one hydrogen atom of the group can be substituted with a substituent. The term "deuterium-substituted or unsubstituted" means that the group to which the term is attached can be an unsubstituted group (a group whose hydrogen atoms are not substituted with substituents) or that at least one hydrogen atom in the group can be a deuterium atom (²H). However, as mentioned above, the hydrogen atom in the molecules of the compounds used in the invention can be either ¹H or ²H (deuterium D). Therefore, the "deuterium substitution" in "deuterium substituted or unsubstituted" is a cautionary note that the term "unsubstituted" does not exclude the manner in which the hydrogen atom is a "deuterium atom". Unless otherwise specified, the "number of carbon atoms" includes the number of carbon atoms of substituents.

In the present description, the "organic layer" means a layer that contains an organic compound in an amount of 70% by weight or more, and the "organic compound" means a compound containing one or more carbon atoms. As the organic compound, employable herein are, for example, those composed only of atoms selected from the group consisting of a carbon atom, a hydrogen atom, a nitrogen atom, an oxygen atom, a sulfur atom, a boron atom, and a halogen atom.

In the present description, "transparent" means that the transmittance of visible light is 50% or more, e.g., 80% or more, e.g., 90% or more, e.g., 99% or more. The visible light transmittance can be measured with a UV/visible light spectrophotometer.

### <Compound represented by general formula (I)>

The compound of the invention is a compound represented by the above general formula (I).

The aromatic ring constituting the "monovalent aromatic hydrocarbon group" of the "substituted or unsubstituted monovalent aromatic hydrocarbon ring" represented by A in the general formula (I), and the aromatic ring constituting the "monovalent aromatic hydrocarbon group" of the "deuterium-substituted or unsubstituted monovalent aromatic hydrocarbon ring" represented by R₁ and R₂ therein can be a single ring, or a fused ring in which two or more rings are fused together, or a linked ring in which two or more rings are linked via a single bond, or a spiro ring in which two or more rings are linked via a spiro bond. In the case of a fused ring, the number of fused rings is preferably 2 to 6, for example, 2 to 4. In the case of a linked ring, the number of linked rings is preferably 2 to 6, more preferably 2 to 4, for example 2, for example 3. The number of the ring skeleton-constituting carbon atoms of the aromatic hydrocarbon group is, for example, 6 to 30, for example, 6 to 22, for example 6 to 18, for example 6 to 14, for example, 6 to 10. Specific examples of the "monovalent aromatic hydrocarbon group" in A, R₁ and R₂ include a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a fluorenyl group, and a spirobifluorenyl group.

The aromatic heterocyclic ring constituting the "monovalent aromatic heterocyclic group" of the "substituted or unsubstituted monovalent aromatic heterocyclic group" represented by A, and the aromatic heterocyclic ring constituting the "monovalent aromatic heterocyclic group" of the "deuterium-substituted or unsubstituted monovalent aromatic heterocyclic group" represented by R₁ and R₂ can be a single ring, or a fused ring in which two or more rings are fused together. In the case of a fused ring, the number of fused rings is preferably 2 to 6, for example, 2 to 4. In the case of a fused ring, only two or more aromatic heterocyclic rings can be fused together, or one or more aromatic heterocyclic rings and one or more aromatic hydrocarbon rings can be fused together. Examples of the heteroatom constituting the aromatic heterocyclic ring include a nitrogen atom, an oxygen atom, and a sulfur atom. The number of the ring skeleton-constituting atoms of the aromatic heterocyclic group is, for example, 4 to 40, for example, 5 to 30, for example, 5 to 20. Specific examples of the "monovalent aromatic heterocyclic group" in A, R₁ and R₂ include a pyridyl group, a pyrimidinyl group, a triazinyl group, a furyl group, a pyrrolyl group, a thienyl group, a quinolyl group, an isoquinolyl group, a benzofuranyl group, benzothienyl group, an indolyl group, a carbazolyl group, a benzoxazolyl group, a benzothiazolyl group, an azafluorenyl group, a diazafluorenyl group, an azaspirofluorenyl group, a diazaspirofluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

For the "substituted or unsubstituted fused polycyclic aromatic group", reference can be made to the description and specific examples of the case of a fused ring formed by fusing two or more rings of the above-mentioned description of "substituted or unsubstituted aromatic hydrocarbon group" and "substituted or unsubstituted aromatic heterocyclic group".

Specific examples of the "substituent" in the case where the "substituted or unsubstituted monovalent aromatic hydrocarbon group" and the "substituted or unsubstituted monovalent aromatic heterocyclic group" represented by A in the general formula (I) each are a substituted aromatic hydrocarbon group and a substituted aromatic heterocyclic group, respectively, include a deuterium atom, a cyano group, a nitro group; a halogen atom such as a fluorine atom, a chlorine atom, a bromine atom, and an iodine atom; a silyl group such as a trimethylsilyl group, and a triphenylsilyl group; a linear or branched alkyl group with 1 to 6 carbon atoms, such as a methyl group, an ethyl group, and a propyl group; a linear or branched alkyloxy group with 1 to 6 carbon atoms, such as a methyloxy group, an ethyloxy group and a propyloxy group; an alkenyl group such as a vinyl group, and an allyl group; an aryloxy group such as a phenyloxy group, and a tolyloxy group; an arylalkyloxy group such as a benzyloxy group, and a phenethyloxy group; a monovalent aromatic hydrocarbon group with 6 to 20 ring skeleton-constituting atoms, such as a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and a monovalent aromatic heterocyclic group with 5 to 20 ring skeleton-constituting atoms, such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; and the hydrogen atoms of these substituents can be further substituted with the substituents exemplified herein. Preferred substituents include a deuterium atom, a linear or branched alkyl group with 1 to 6 carbon atoms, and a monovalent aromatic hydrocarbon group with 6 to 20 ring skeleton-constituting atoms. As for substituents substituted with substituents, the substituents directly substituted on the parent skeleton (aromatic hydrocarbon group, aromatic heterocyclic group) can be referred to as "first substituents" and those substituted on the first substituents as "second substituents". Here, when the first substituent contains a benzene ring, the benzene ring can bond to the parent skeleton to form a ring structure. When two or more substituents are substituted on the benzene ring of the first substituent, adjacent substituents can bond to each other to form a ring structure. Here, the bond between the benzene ring and the parent skeleton in the first substituent and the bond between the second substituents can be a single bond or a bond via a linking group. Examples of the linking group include a substituted or unsubstituted methylene group, an oxygen atom, and a sulfur atom.

For the description of the aromatic ring constituting the "divalent aromatic hydrocarbon group" of the "deuterium-substituted or unsubstituted divalent aromatic hydrocarbon ring" and the aromatic heterocyclic ring constituting the "divalent aromatic heterocyclic group" of the "deuterium-substituted or unsubstituted divalent aromatic heterocyclic ring" in L₁, L₂ and L₃ in the general formula (I), reference can be made to the description of the aromatic ring constituting the "monovalent aromatic hydrocarbon group" and the aromatic heterocyclic ring constituting the "monovalent aromatic heterocyclic group" in A, R₁ and R₂. Specific examples of the "divalent aromatic hydrocarbon group" include divalent groups formed by removing one hydrogen atom from the specific examples of the "monovalent aromatic heterocyclic group" mentioned above, and specific examples of the "divalent aromatic heterocyclic group" include divalent groups formed by removing one hydrogen atom from the specific examples of the "monovalent aromatic heterocyclic group" mentioned above.

The position of the bond of the "naphthylene group" of the "deuterium-substituted or unsubstituted naphthylene group" represented by B and C is not particularly limited. Examples of the naphthylene group include a 1,2-naphthylene group, a 1,3-naphthylene group, a 1,4-naphthylene group, a 2,4-naphthylene group, a 2,5-naphthylene group, a 2,6-naphthylene group, and a 2,8-naphthylene group.

As L₁ and L₂ in the general formula (I), preferred is a deuterium-substituted or unsubstituted divalent aromatic hydrocarbon group, more preferred is a deuterium-substituted or unsubstituted biphenylene group, or a deuterium-substituted or unsubstituted phenylene group, even more preferred is a phenylene group, further more preferred is a 1,4-phenylene group. L₃ in the general formula (I) is more preferably a single bond or a substituted or unsubstituted phenylene group, for example, a single bond, and for example, a phenylene group (preferably a 1,3-phenylene group, a 1,4-phenylene group, more preferably a 1,4-phenylene group). When A-L₃ is a group with a structure with two or more aromatic rings linking to each other, L₃ is a substituted or unsubstituted phenylene group.

A in the general formula (I) is preferably a substituted or unsubstituted monovalent aromatic hydrocarbon group, more preferably a substituted or unsubstituted phenyl group, a substituted or unsubstituted naphthyl group, a substituted or unsubstituted dibenzofuranyl group, a substituted or unsubstituted dibenzothienyl group, a substituted or unsubstituted phenanthrenyl group, or a substituted or unsubstituted biphenyl group, even more preferably a substituted or unsubstituted phenyl group, a substituted or unsubstituted 9-phenanthrenyl group.

B and C in the general formula (I) are preferably a 1,2-naphthylene group, a 1,3-naphthylene group, a 2,4-naphthylene group, or a 2,8-naphthylene group.

R₁ and R₂ in the general formula (I) are preferably a deuterium-substituted or unsubstituted phenyl group, a deuterium-substituted or unsubstituted naphthyl group, a deuterium-substituted or unsubstituted dibenzofuranyl group, a deuterium-substituted or unsubstituted phenanthrenyl group, or a deuterium-substituted or unsubstituted biphenyl group, more preferably a phenyl group or a naphthyl group.

As a preferred compound group represented by the general formula (I), here can be shown a compound group 1 where R₁-B-L₁ and R₂-C-L₂ are different groups. The compound group 1 includes a compound group 1a wherein L₃ is a single bond and A is a substituted or unsubstituted aromatic hydrocarbon group; a compound group 1b wherein L₃ is a deuterium-substituted or unsubstituted aromatic hydrocarbon group (e.g., a deuterium-substituted or unsubstituted phenylene group) and A is a substituted or unsubstituted aromatic hydrocarbon group; and a compound group 1c wherein L₃ is a deuterium-substituted or unsubstituted aromatic hydrocarbon group (e.g., a deuterium-substituted or unsubstituted phenylene group) and A is a substituted or unsubstituted aromatic heterocyclic group. The compound groups 1a to 1c can each have the same R₁ and R₂, the same L₁ and L₂, and different B and C. The compound groups 1a to 1c can each have the same B and C, the same L₁ and L₂, and different R₁ and R₂. The compound groups 1a to 1c can each have the same L₁ and L₂, different R₁ and R₂, and different B and C. A in the compound groups 1a and 1b can be a phenyl group that can be substituted with one or more groups selected from the group consisting of phenyl, naphthyl and phenanthrenyl groups. A in the compound group 1c can be a dibenzofuranyl or dibenzothienyl group. The compound groups 1a to 1c can each further satisfy at least one of the following additional conditions.

One additional condition is that L₁ and L₂ each are a deuterium-substituted or unsubstituted divalent aromatic hydrocarbon group, such as a phenylene group. One additional condition is that B and C each are a deuterium substituted or unsubstituted 1,2-naphthylene group, a deuterium substituted or unsubstituted 1,3-naphthylene group, or a deuterium substituted, or unsubstituted 1,4-naphthylene group. One additional condition is that B and C each are a deuterium substituted or unsubstituted 2,4-naphthylene group, a deuterium substituted or unsubstituted 2,5-naphthylene group, a deuterium substituted or unsubstituted 2,6-naphthylene group, or a deuterium substituted or unsubstituted 2,8-naphthylene group. One additional condition is that R₁ and R₂ each are a deuterium-substituted or unsubstituted monovalent aromatic hydrocarbon group, for example, a phenyl group optionally substituted with one or more groups selected from the group consisting of phenyl, naphthyl and phenanthrenyl groups. One additional condition is that R₁ and R₂ each are a deuterium-substituted or unsubstituted monovalent aromatic heterocyclic group, for example, a dibenzofuranyl or dibenzothienyl group.

As a preferred compound group represented by the general formula (I), here can be shown a compound group 2 where R₁-B-L₁ and R₂-C-L₂ are the same groups. The compound group 2 includes a compound group 2a wherein L₃ is a single bond and A is a substituted or unsubstituted aromatic hydrocarbon group; a compound group 2b wherein L₃ is a deuterium-substituted or unsubstituted aromatic hydrocarbon group (e.g., a deuterium-substituted or unsubstituted phenylene group) and A is a substituted or unsubstituted aromatic hydrocarbon group; and a compound group 2c wherein L₃ is a deuterium-substituted or unsubstituted aromatic hydrocarbon group (e.g., a deuterium-substituted or unsubstituted phenylene group) and A is a substituted or unsubstituted aromatic heterocyclic group. Ain the compound groups 2a and 2b can be a phenyl group that can be substituted with one or more groups selected from the group consisting of phenyl, naphthyl and phenanthrenyl groups. A in the compound group 2c can be a dibenzofuranyl or dibenzothienyl group. The compound groups 2a to 2c can each further satisfy at least one of the additional conditions described for the compound group 1.

The molecular weight of the compound represented by the general formula (I) is preferably 2000 or less, more preferably 1500 or less, and can be, for example, 1000 or less. The lower limit of the molecular weight of the compound represented by the general formula (I) is a molecular weight of the molecule having a smallest molecule that the general formula (I) can take.

Specific examples of the compound represented by the general formula (I) are shown below. However, the compound represented by the general formula (I) employable in the invention should not be limitatively interpreted by these specific examples. In the chemical structural formulae below, the hydrogen atom (¹H) is omitted and the deuterium atom (²H) is indicated as "D".

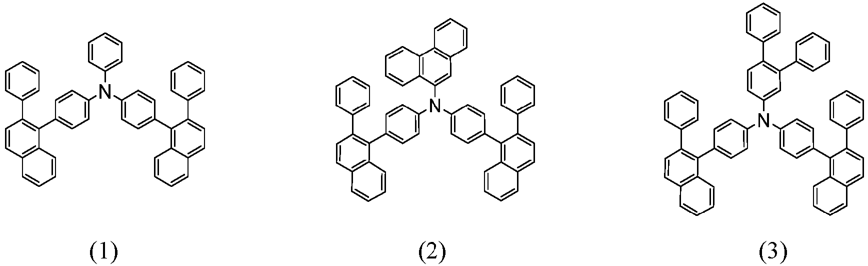

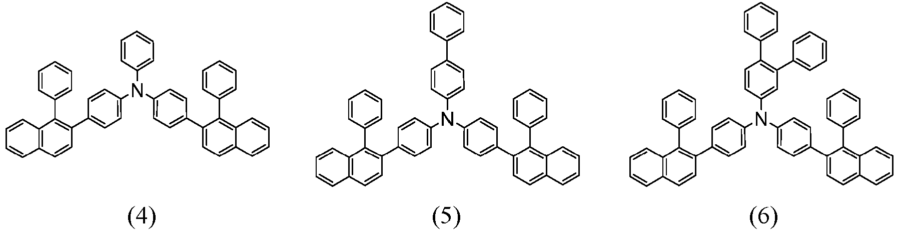

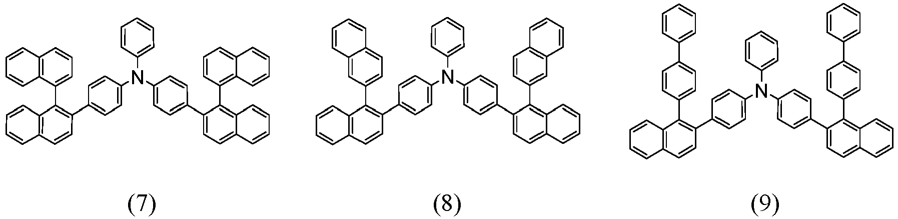

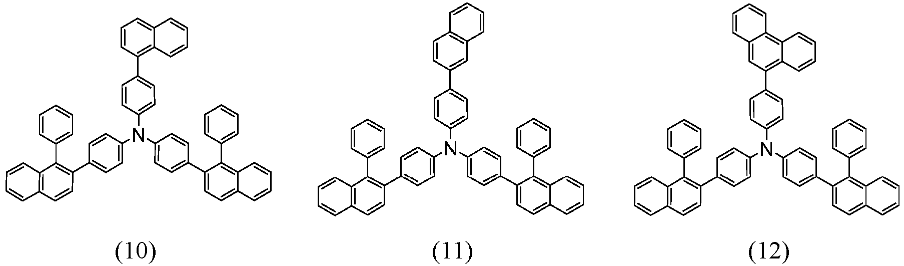

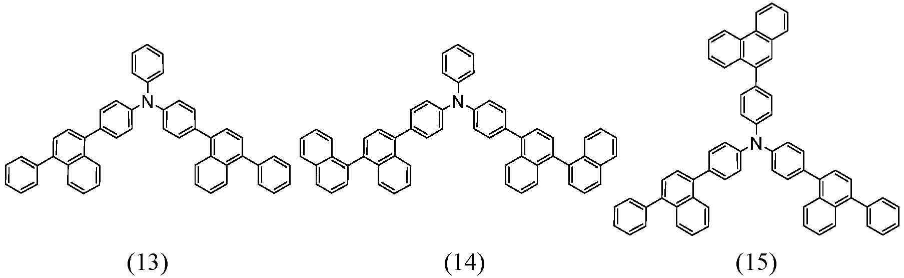

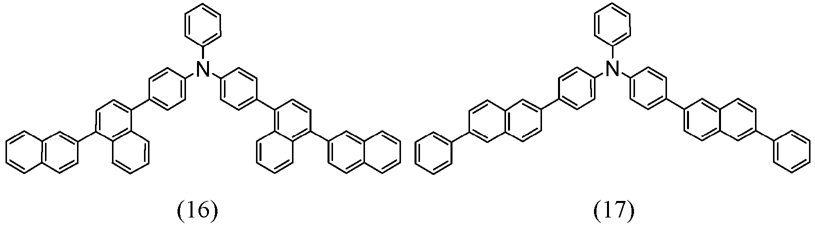

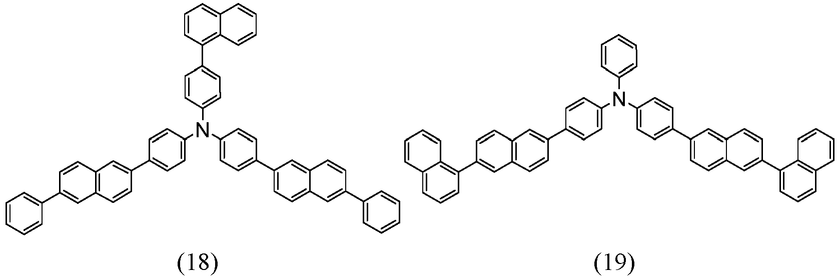

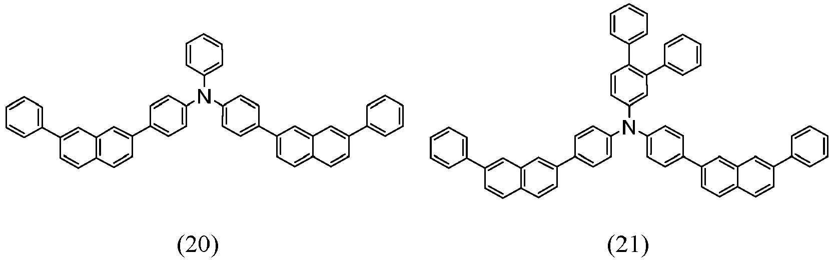

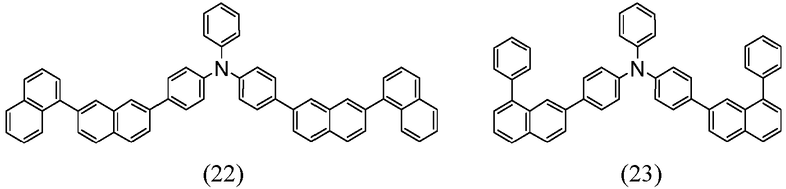

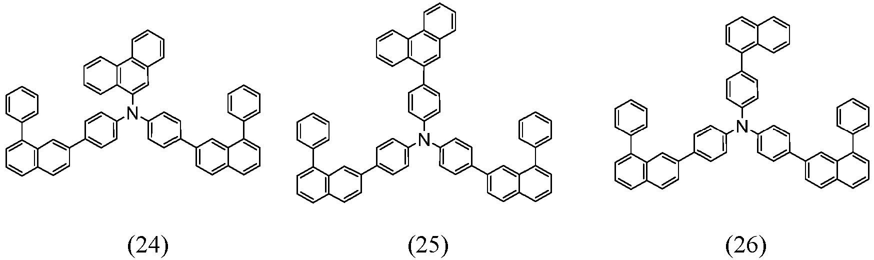

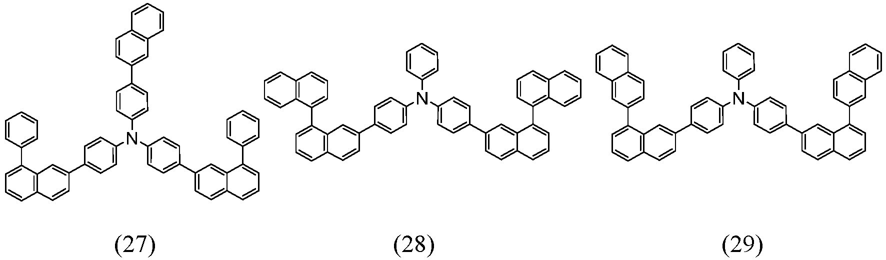

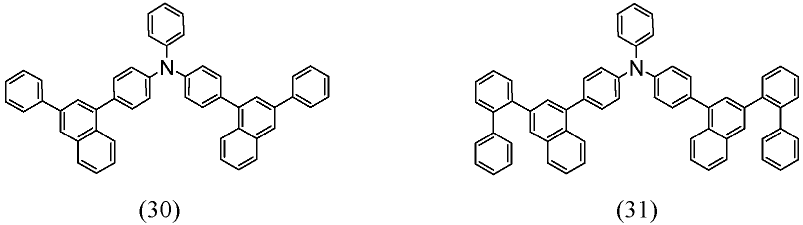

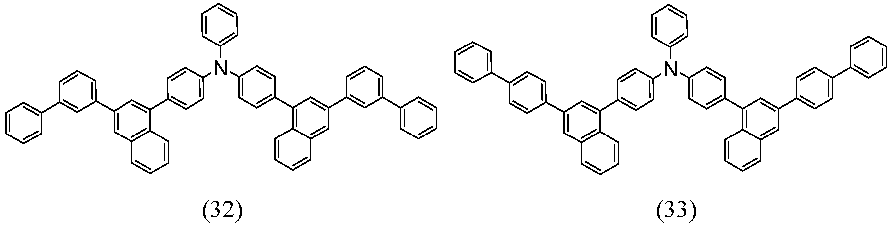

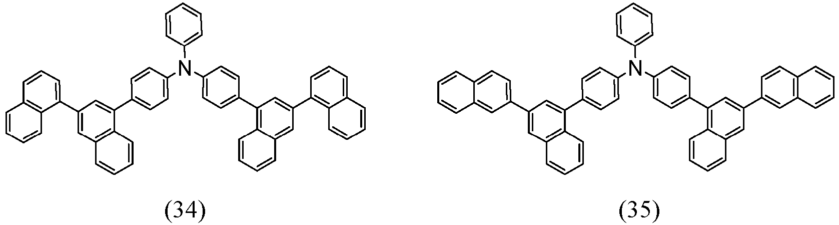

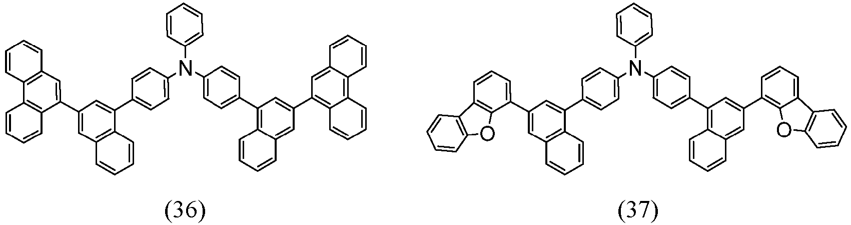

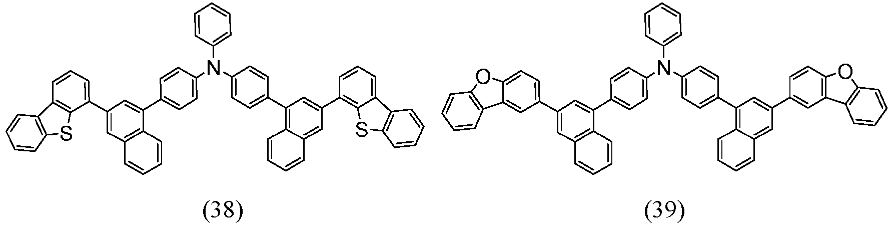

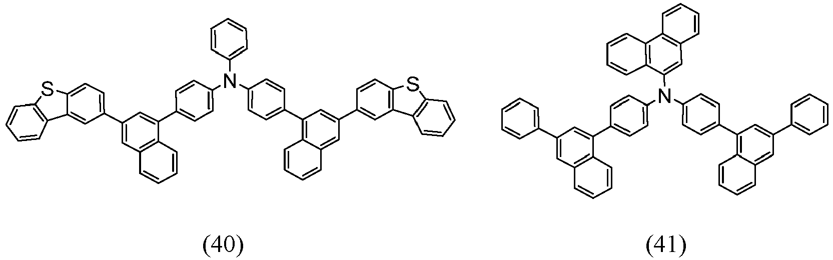

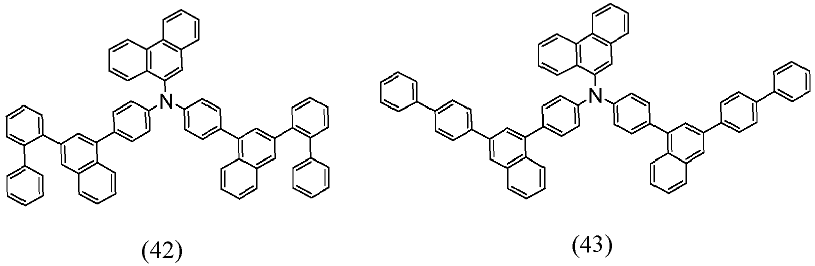

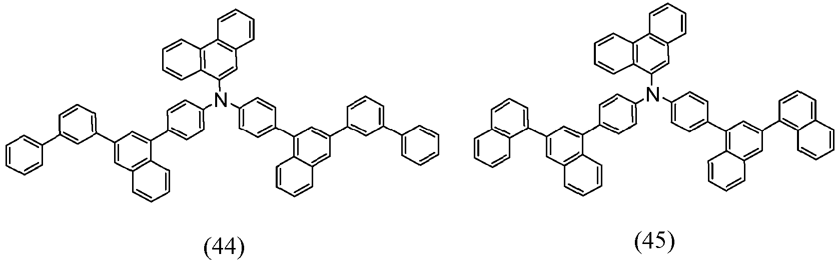

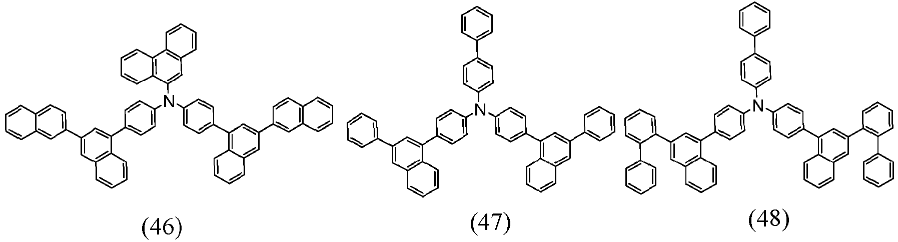

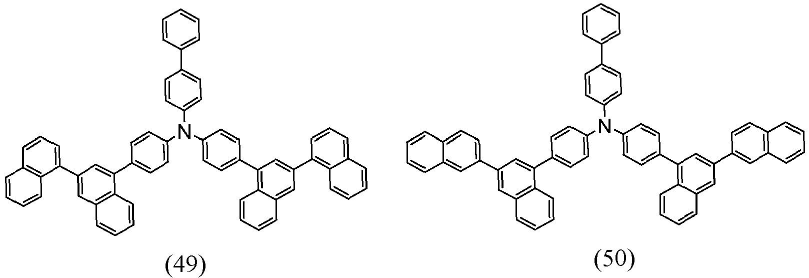

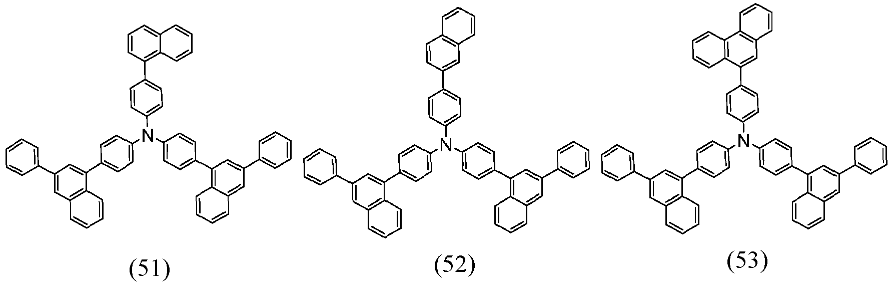

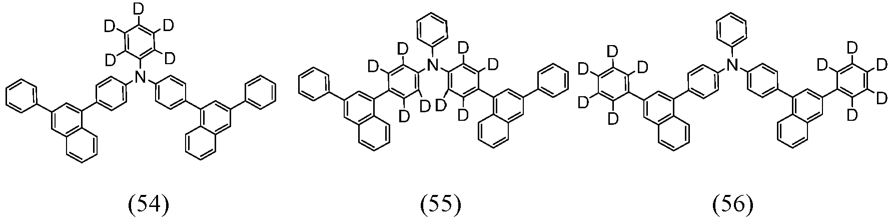

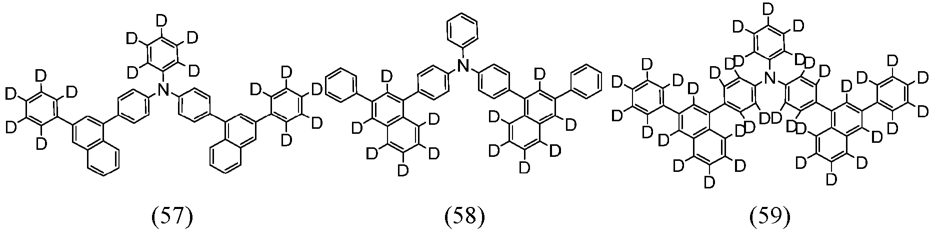

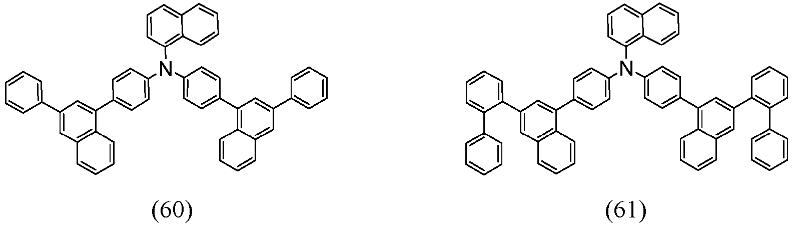

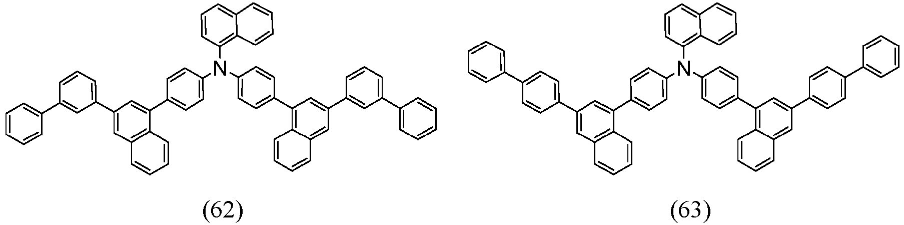

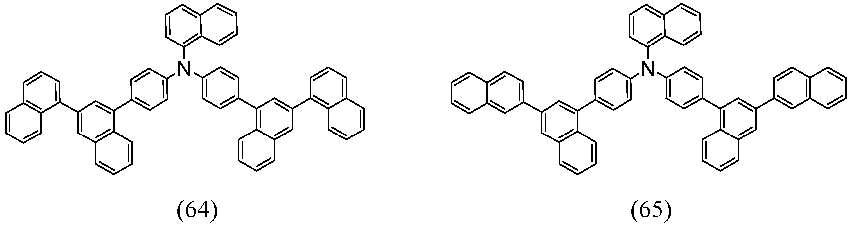

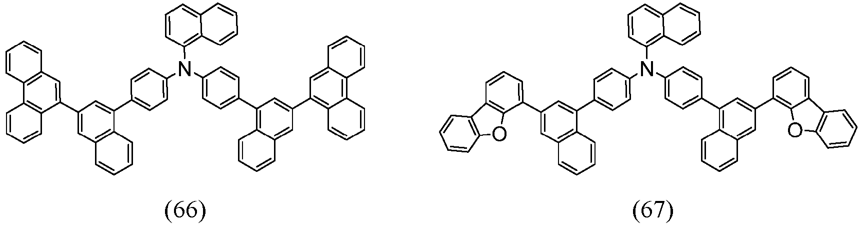

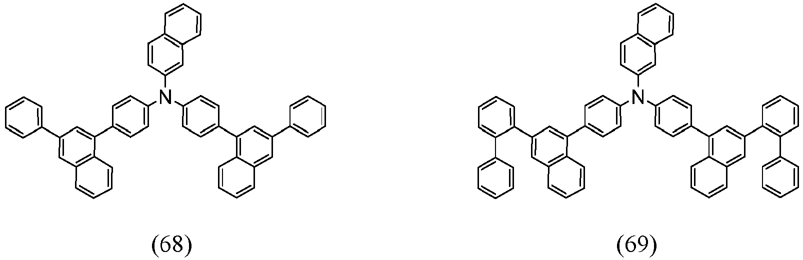

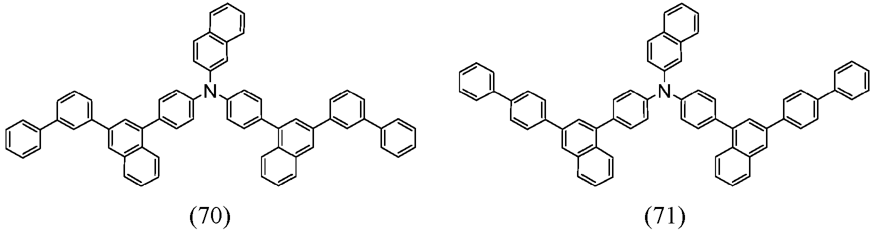

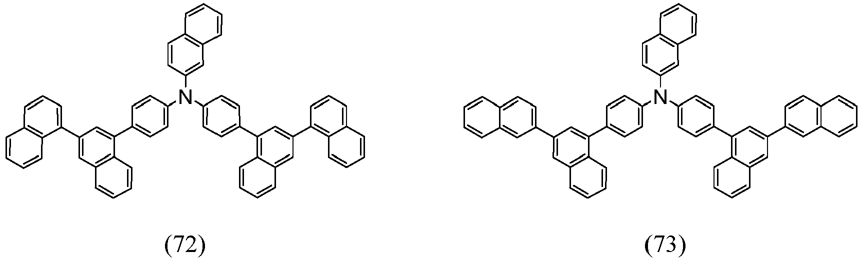

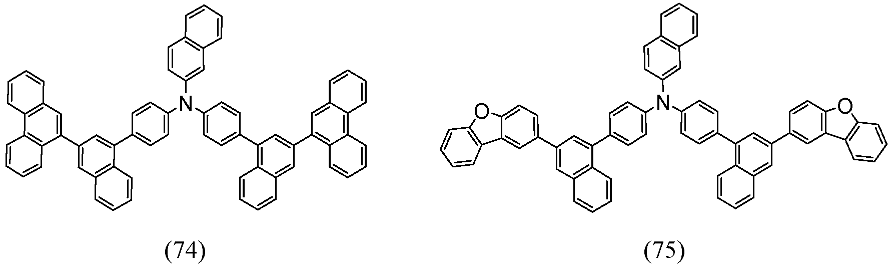

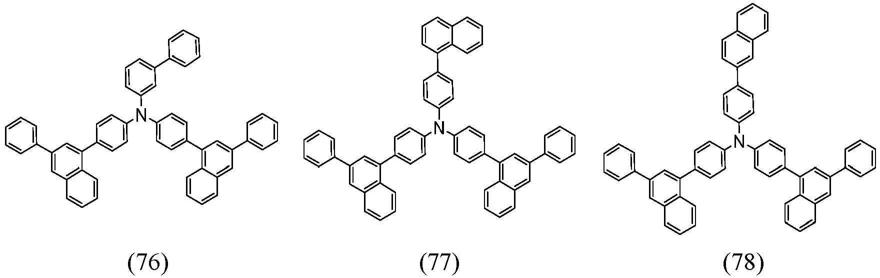

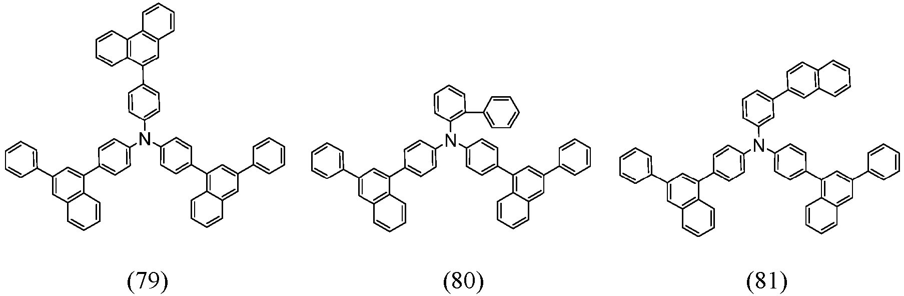

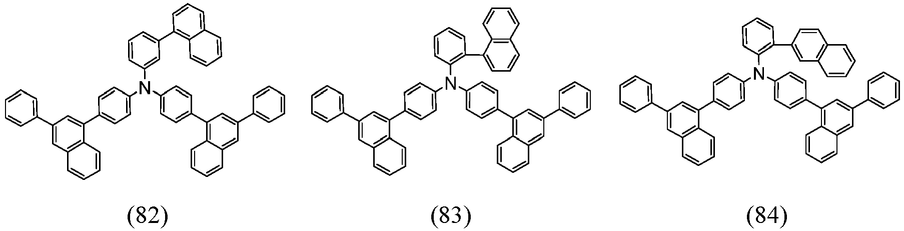

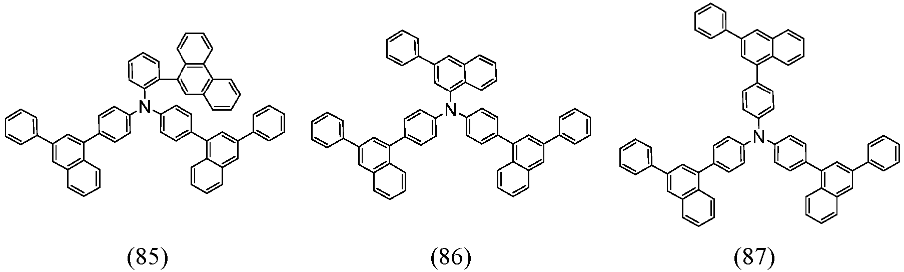

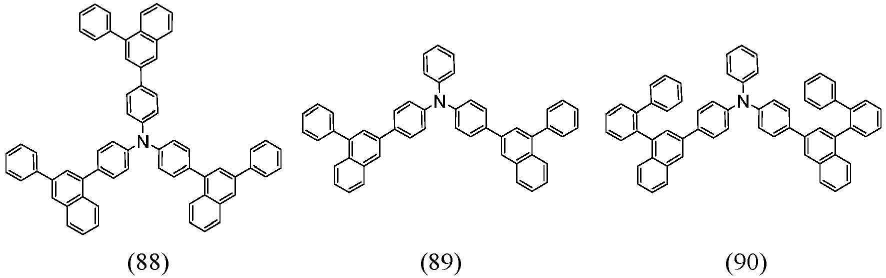

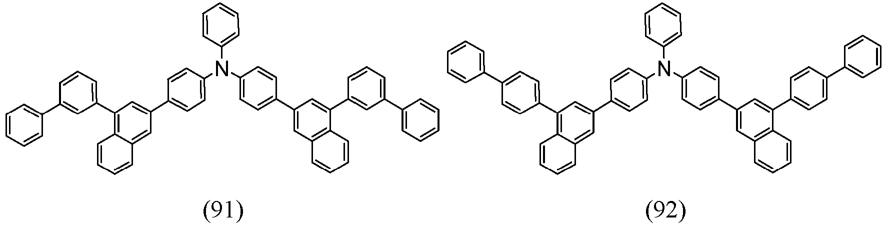

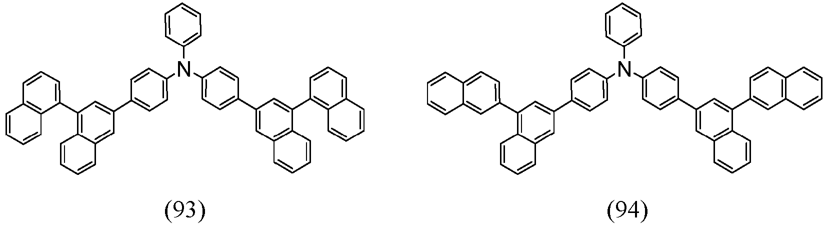

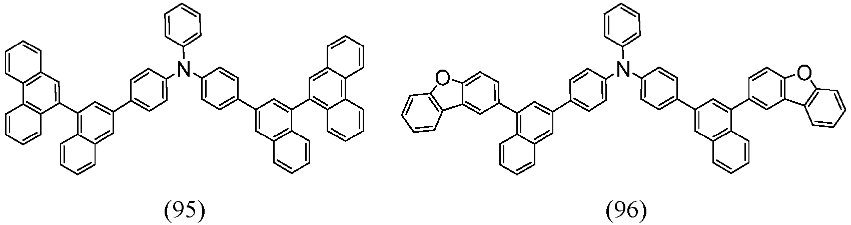

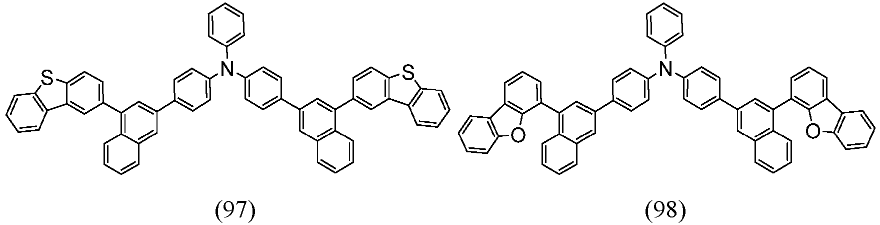

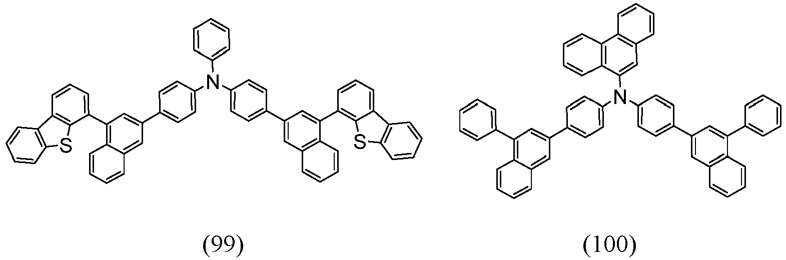

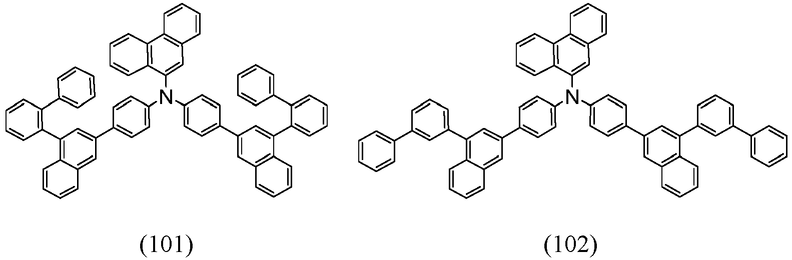

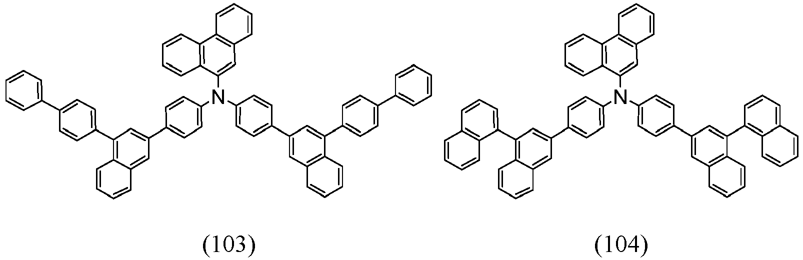

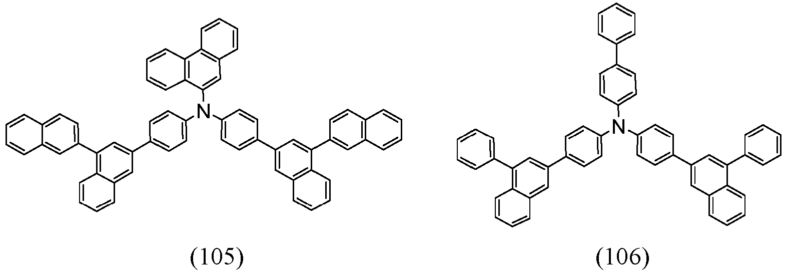

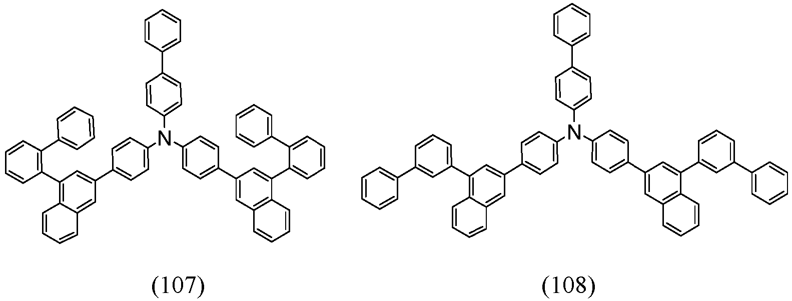

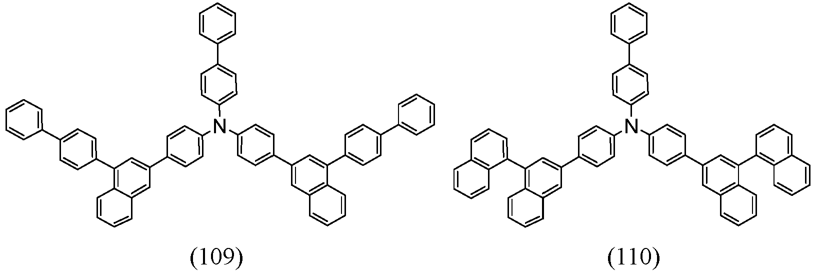

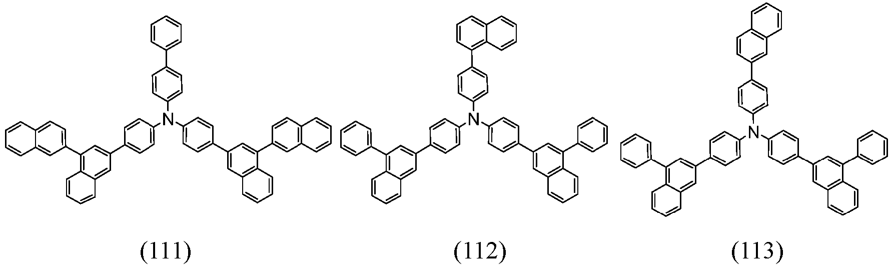

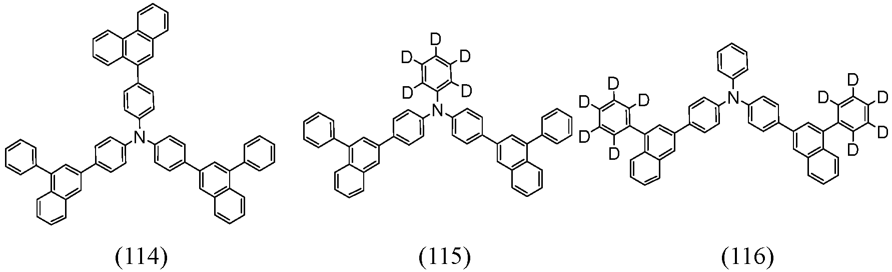

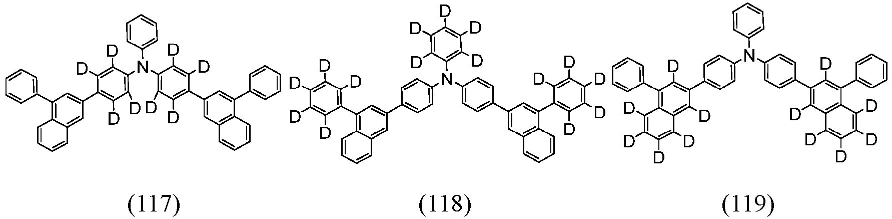

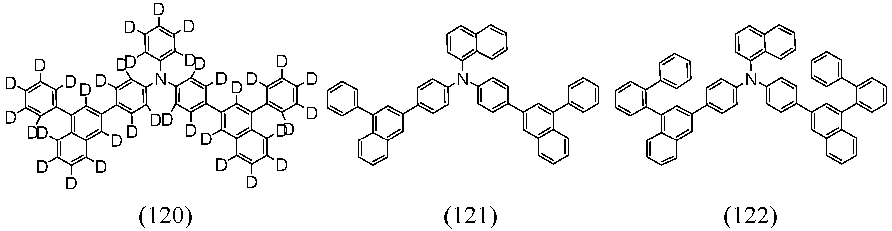

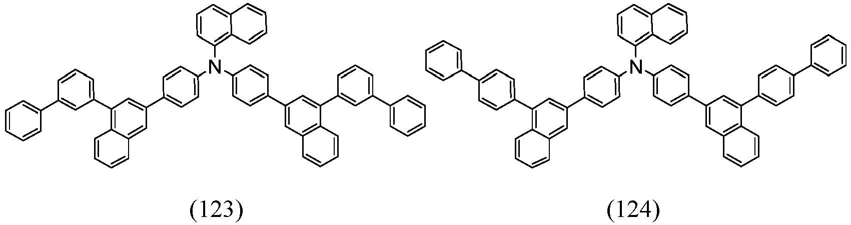

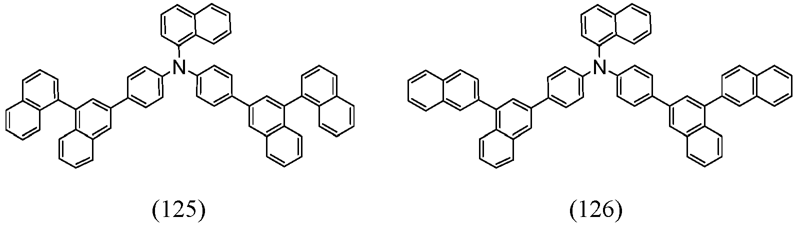

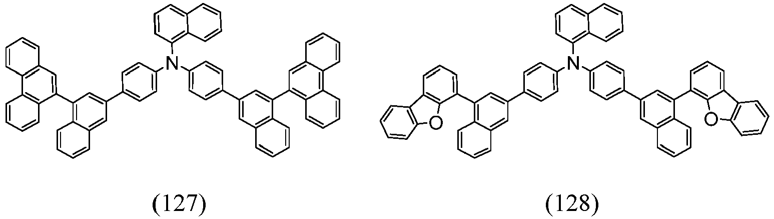

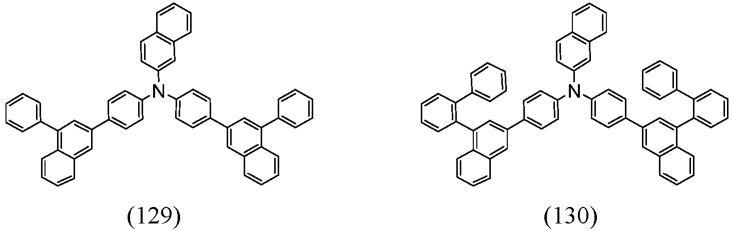

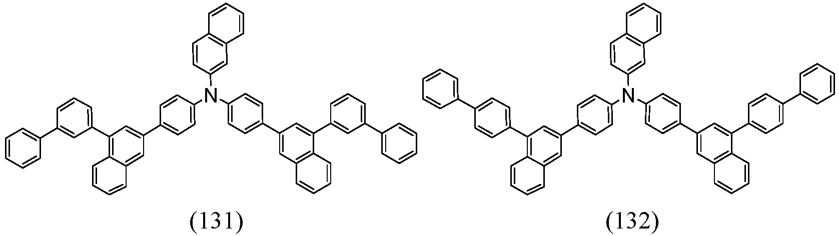

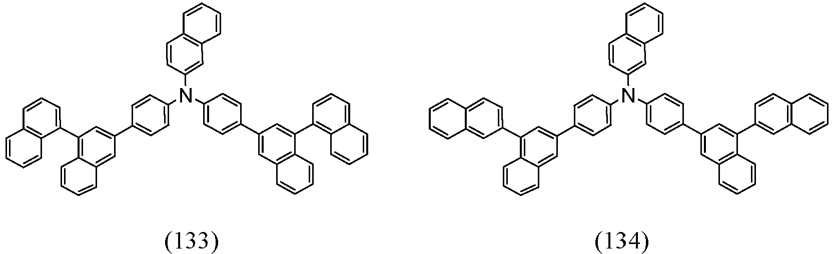

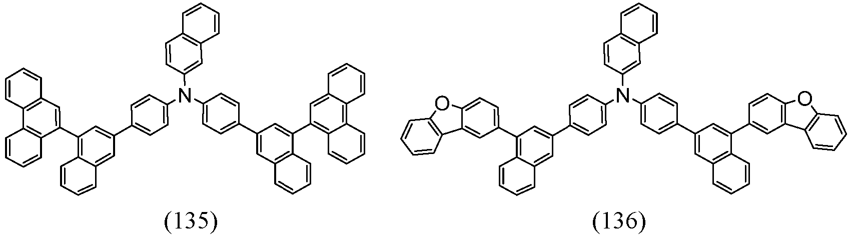

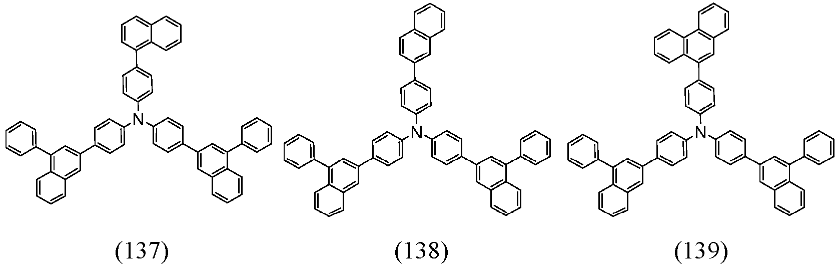

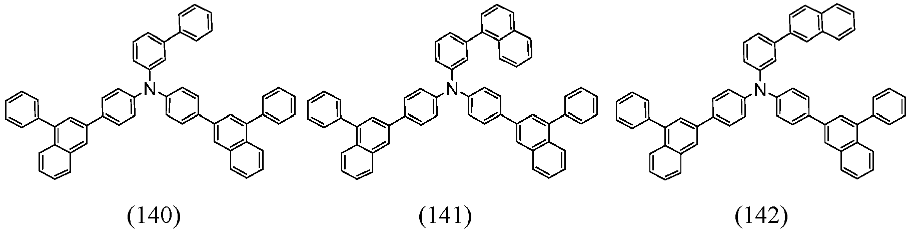

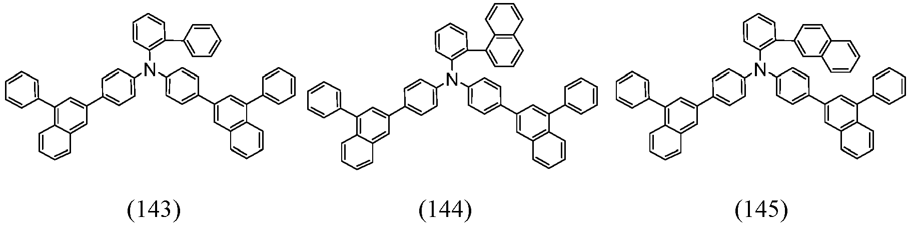

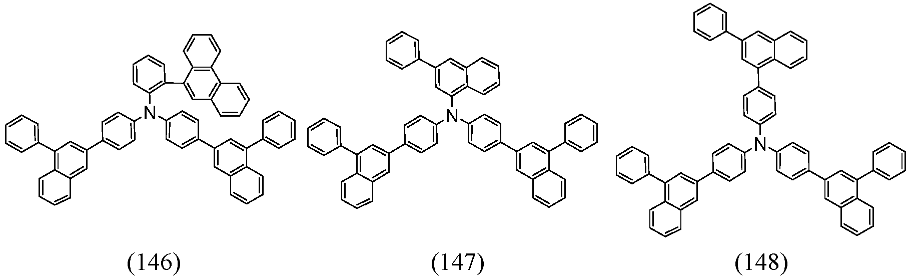

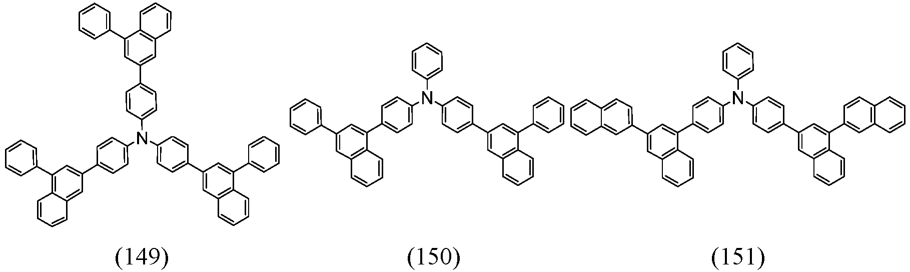

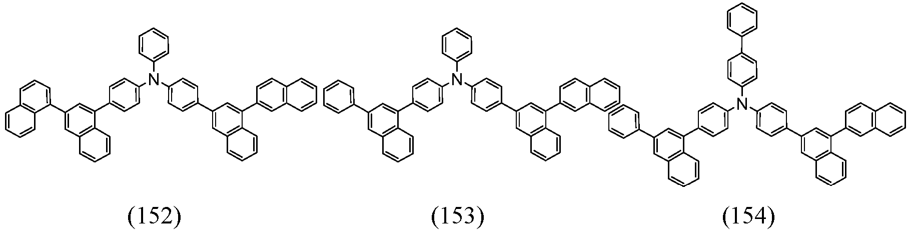

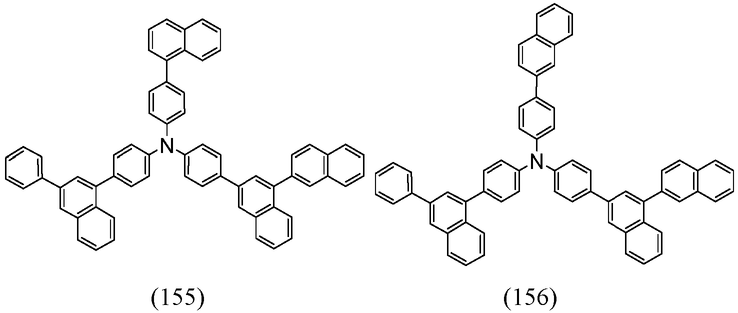

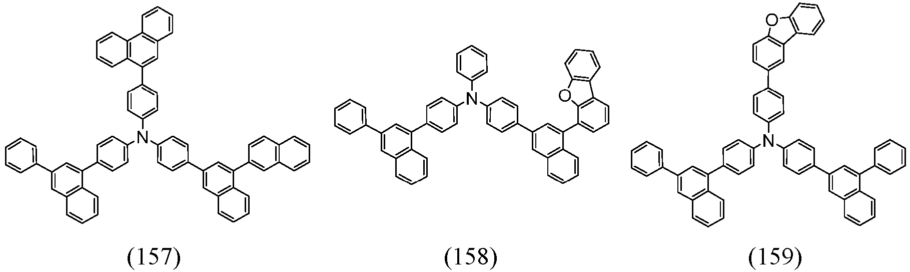

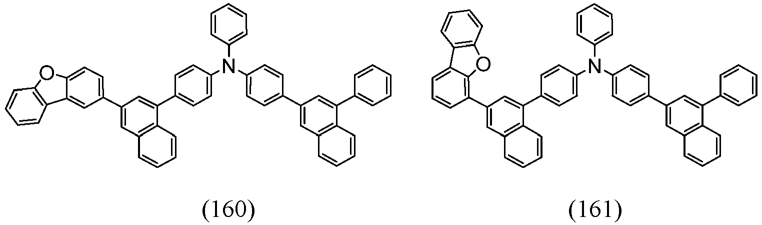

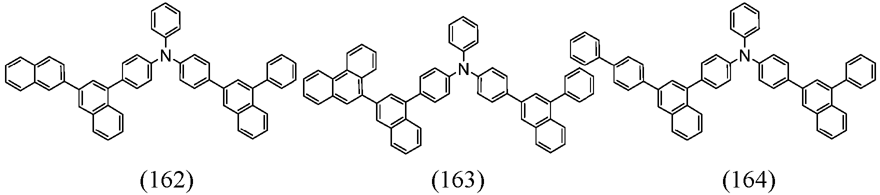

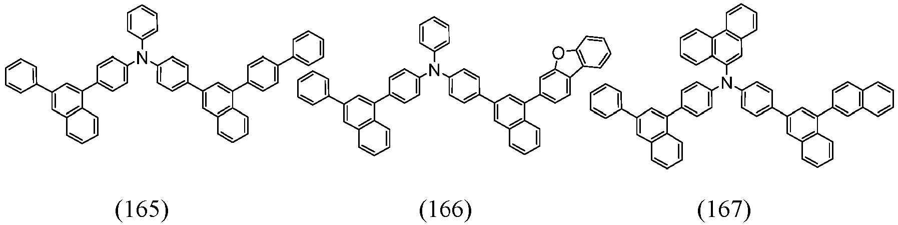

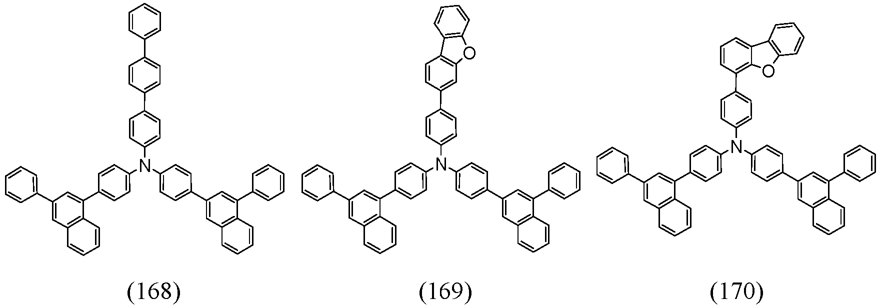

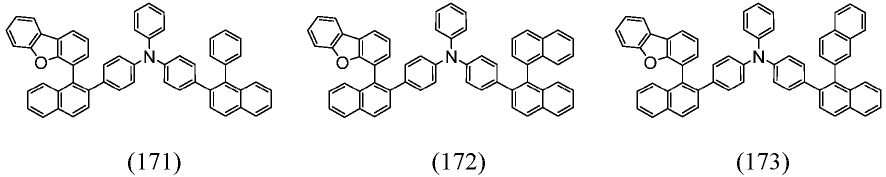

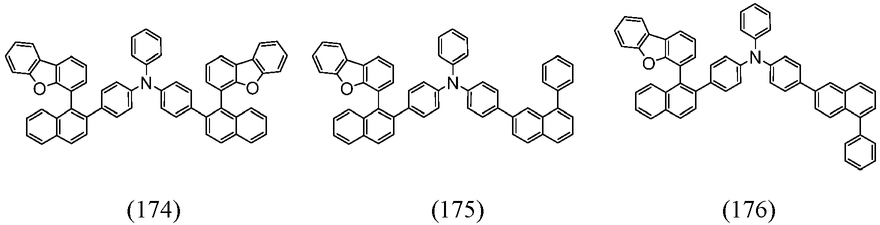

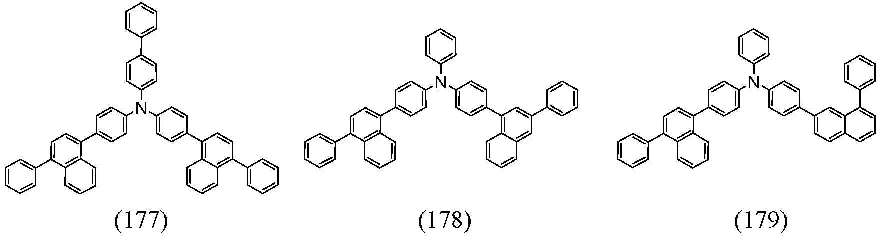

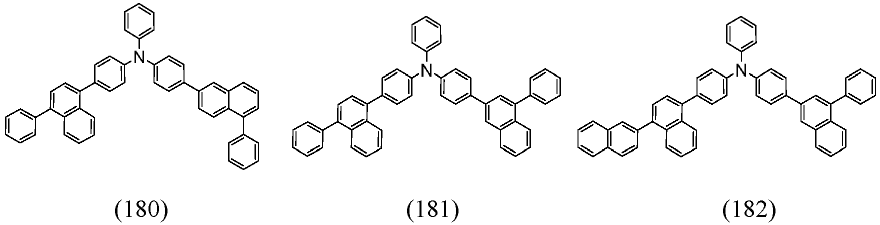

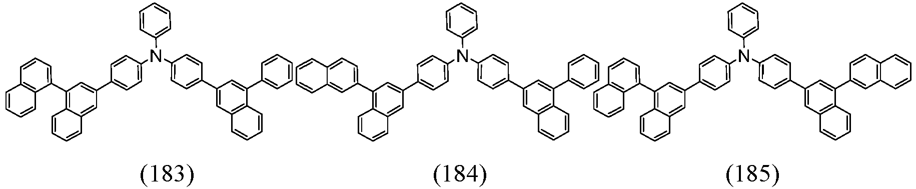

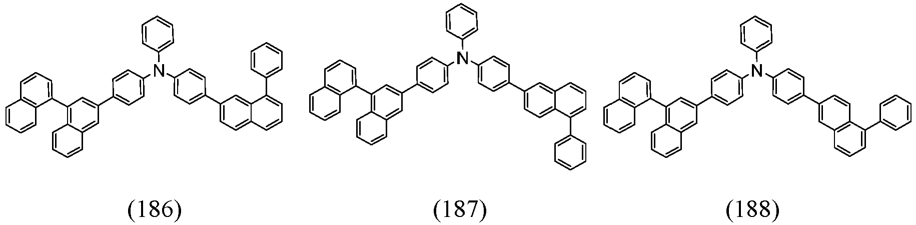

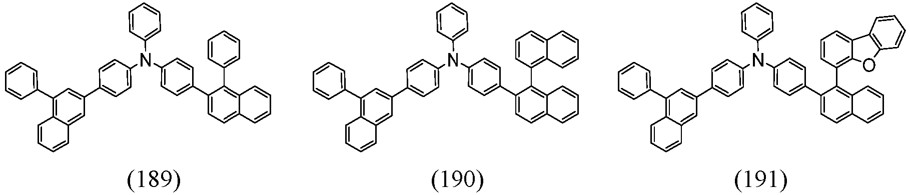

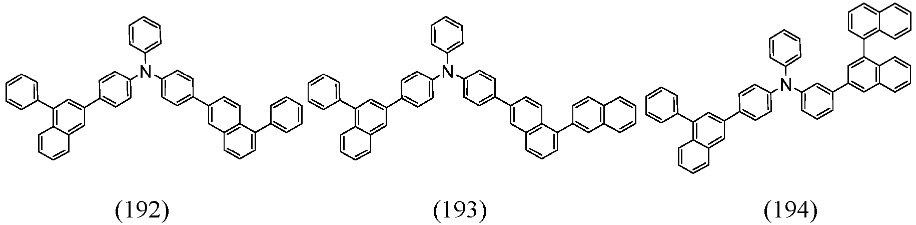

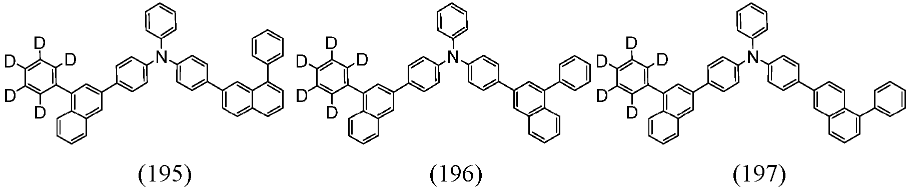

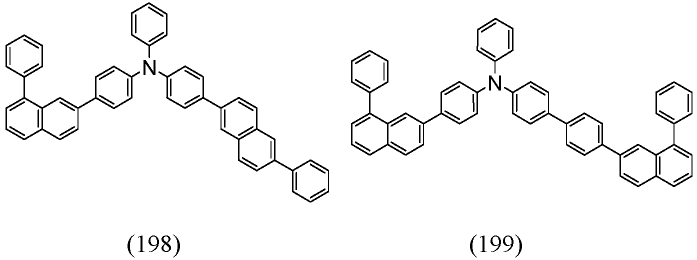

### [Usefulness of compound represented by general formula (I)]

The compound represented by the general formula (I) of the invention has excellent hole transport and electron blocking abilities and high thermal stability in the thin film state, making it useful as a material for organic layers in organic EL devices. The compound represented by the general formula (I) can be used, for example, as constituent materials for the electron blocking layer, hole transporting layer, and light emitting layer of organic EL devices, and are particularly useful as constituent materials for the electron blocking layer and hole transporting layer. An organic EL device containing the compound represented by the general formula (I) in the organic layers (e.g., at least one of the electron blocking layer, hole transporting layer, and light emitting layer) can realize a low drive voltage, a high luminous efficiency, and a long device lifetime.

### [Synthesis Method and Characteristics Evaluation Method for Compound Represented by General Formula (I)]

The compound represented by the general formula (I) is a novel compound.

The Compound represented by the general formula (I) can be synthesized by applying known coupling reactions and selecting known reaction conditions as appropriate. For details of the reactions, Synthesis Examples described later can be referred to.

The compound represented by the general formula (I) can be purified by known methods, such as purification by column chromatography, adsorption purification using silica gel, activated charcoal, or activated clay, or solvent recrystallization, crystallization, or sublimation purification. Compounds can be identified by NMR analysis.

Physical properties that serve as indicators of the usefulness of the compound represented by the general formula (I) include melting point, glass transition point (Tg) and energy level of HOMO. The melting point is an indicator of deposition properties, the glass transition point (Tg) is an indicator of the stability in a thin film state, and the HOMO energy level is an indicator of hole injection, hole transport, or electron blocking properties.

The melting point and glass transition point (Tg) can be measured, for example, with a high-sensitivity differential scanning calorimeter (DSC 3100SA, manufactured by Bruker AXS) using a powder of the compound to be measured.

The HOMO energy level can be determined, for example, by preparing a thin film of the compound to be measured with a thickness of 100 nm on a substrate with ITO and using an ionization potential measurement system (PYS-202, manufactured by Sumitomo Heavy Industries, Ltd.).

### <Organic Electroluminescent Device>

The organic electroluminescent device (organic EL device) of the invention has a pair of electrodes, organic layers disposed between the pair of electrodes and including at least a light emitting layer, and at least one of the organic layers contains the compound represented by the general formula (I). Here, the pair of electrodes are an anode and a cathode. For the description of the compound represented by the general formula (I), reference can be made to the description in the above section of <Compound represented by general formula (I)>. The compounds represented by the general formula (I) have excellent hole transport and electron blocking abilities and high thermal stability in the thin film state, so using them as materials for organic layers can realize organic EL devices with a low drive voltage, a high luminous efficiency, and a long device lifetime.

The organic layers that the organic EL device of the invention has include at least one light emitting layer, and the organic layers of the device can be light emitting layers alone, or the device can have one or more organic layers in addition to a light emitting layer. Here, the compound represented by the general formula (I) can be contained in the light emitting layer, or can be contained in any other organic layer than the light emitting layer, but is preferably contained in the organic layer disposed between the light emitting layer and the anode. Specific examples of organic layers to be disposed between the light emitting layer and the anode include a hole injection layer, a hole transporting layer, and an electron blocking layer; and the compound represented by the general formula (I) is preferably contained in the hole transporting layer and electron blocking layer, and is more preferably contained in the electron blocking layer. On the other hand, specific examples of the organic layers to be disposed between the light emitting layer and the cathode include a hole blocking layer, an electron transporting layer, and an electron injection layer. The materials for these organic layers can be appropriately selected from known materials.

In one aspect of the invention, the organic EL device has a laminate structure in which an anode, a hole injection layer, a hole transporting layer, an electron blocking layer, a light emitting layer, an electron transporting layer, an electron injection layer, and a cathode are laminated in order on a substrate, and at least the electron blocking layer contains the compound represented by the general formula (I). Here, the device can have a hole blocking layer between the light emitting layer and the electron transporting layer. Also, in the organic EL device, one organic layer can have two or more roles in combination. Examples of such organic layers include a hole injection transport layer that serves as both a hole injection layer and a hole transporting layer, and an electron injection transport layer that serves as both an electron injection layer and an electron transporting layer. The organic EL device can also have a laminate structure in which two or more organic layers having the same function are laminated. For example, such laminate layers of the type include a laminate configuration of two hole transporting layers, a laminate configuration of two light emitting layers, and a laminate configuration of two electron transporting layers.

In the following, the constituent members and layers of the organic EL device are described in detail.

### [Anode]

Electrode materials with a large work function, such as ITO and gold, are used for the anode in the organic EL device of the invention.

### [Hole Injection Layer, hole transporting layer]

The hole injection layer is provided between the anode and the light emitting layer or between the anode and the hole transporting layer to lower the injection barrier of holes supplied from the anode, thereby lowering the drive voltage and improving the emission intensity.

The hole transporting layer is a layer that functions to transport holes. The hole transporting layer may be a hole injection transport layer that also functions as a hole injection layer.

Compounds represented by the general formula (I) or general formula (II) can be used as materials for the hole transporting layer and hole injection transport layer. By using compounds represented by the general formula (I) or general formula (II) as materials for the hole transporting layer, EL devices that exhibit a low drive voltage and a high luminous efficiency and have a long device lifetime can be realized. The compound represented by the general formula (I) or general formula (II) used in the hole transporting layer and hole injection transport layer may be one or more of the group of the compounds represented by the general formula (I) and general formula (II). The compound represented by the general formula (I) or general formula (II) may be also used in combination with any other hole transporting material or hole injection material. For the details and the preferred range of the compound represented by the general formula (II), reference can be made to the corresponding description of [0052] to [0059] in PTL 8. Also, specific examples of the compound represented by the general formula (II) include compounds (1-1) to (1-43) described in [0108] to [0150] of PTL 8, cited herein as part of this description. In the organic EL device of the invention, it is preferable to form a layer containing the compound represented by the general formula (I) as a layer adjacent to the layer containing the compound represented by the general formula (II), and it is more preferred to form an electron blocking layer containing the compound of the general formula (I) as a layer adjacent to the hole transporting layer containing the compound of the general formula (II). In the invention, preferably, the compound represented by the general formula (II) can be used in the hole transporting layer, and along with it, the compound represented by the general formula (II) can also be used in the hole injection layer adj acent thereto. The compound of the general formula (II) used for the hole injection layer may be different from or the same as the compound of the general formula (II) used for the hole transporting layer. When the same compound is used, preferably, the compound of the general formula (II) is mixed with any other hole injection material for the hole injection layer.

As materials for the hole injection layer, porphyrin compounds such as copper phthalocyanine, starburst-type triphenylamine derivatives, arylamine compounds having two or more triphenylamine or carbazolyl structures in the molecule, each of which is linked by a single bond or a divalent group that does not contain a heteroatom, acceptor heterocyclic compounds such as hexacyanoazatriphenylene, and coating-type polymer materials, can be used.

As materials for the hole injection layer, the hole transporting layer and the hole injection transport layer, in addition to the compounds represented by the general formula (I),benzidine derivatives such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylbenzidine (NPD), 1,1-bis[(di-4-tolylamino)phenyl]cyclohexane (TAPC), and arylamine compounds having two or more triphenylamine or carbazolyl structures in the molecule , each of which is linked by a single bond or a divalent group that does not contain a heteroatom, can be used. Coating-type polymer materials, such as poly(3,4-ethylenedioxythiophene) (PEDOT)/poly(styrenesulfonate) (PSS), and polymer compounds with a benzidine derivative structure as a partial structure thereof, such as TPD, can also be used.

These materials can be formed as a single film consisting of one type of the material, or as a mixed film consisting of plural types thereof. The hole transporting layer may be a monolayer structure of a single film or a mixed film, a laminate structure of multiple layers of a single film, a laminate structure formed of multiple layers of a mixed film, or also a laminate structure of one or more single layers and one or more mixed layers.

The hole injection and hole transporting layers may also be formed by adding a P-type dopant such as tris-bromophenylamine hexachloroantimony or a radialene derivative described in EP 2684932 to the hole injection or hole transporting materials.

The absolute value of the HOMO energy level of the hole transporting material is preferably larger (i.e., having a deeper HOMO level) than the absolute value of the HOMO level of general hole transporting materials such as NPD and TPD (5.4 eV), and is preferably smaller than the absolute value of the HOMO energy level of the electron blocking material described below. Deeper HOMO levels mean better hole transport ability. On the other hand, when the absolute value of the HOMO energy level of the hole transporting material is smaller than the absolute value of the HOMO level of the electron blocking material, the transport of holes to the light emitting layer is hindered. Specifically, the absolute value of the HOMO energy level of the hole transporting material is preferably 5.45 eV or more and 5.80 eV or less, more preferably 5.50 eV or more and 5.65 eV or less.

### [Electron Blocking Layer]

The electron blocking layer is placed, for example, between the light emitting layer and the hole transporting layer and has the function of suppressing the diffusion of electrons in the light emitting layer to the outside of the light emitting layer (toward the hole transporting layer). This improves the recombination probability of electrons and holes in the light emitting layer. Usually, the electron blocking layer additionally has the function of transporting holes. The electron blocking layer may also serve as an exciton blocking layer that suppresses the diffusion of excitons from the light emitting layer.

The compound represented by the general formula (I) can be used as a material for the electron blocking layer. The compound represented by the general formula (I) has excellent electron blocking ability, high electron tolerance, and stability in the thin film state, as well as the feature of confining excitons generated in the light emitting layer. Thereby, the organic EL device using the compound represented by the general formula (I) as an electron-blocking material has high luminous efficiency because the probability of recombination between holes and electrons is improved and thermal deactivation is suppressed, and accordingly, the maximum luminous intensity is improved because the drive voltage is lowered and the current resistance is improved. The compound represented by the general formula (I) used in the electron blocking layer may be one or more of the compounds in the group of compounds represented by the general formula (I). The compound represented by the general formula (I) may be used in combination with other electron blocking materials.

As materials for the electron blocking layer, in addition to the compound represented by the general formula (I), carbazole derivatives, such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz), and compounds having electron blocking effect such as compounds having a triphenylsilyl group and a triarylamine structure, as typified by 9-[4-(carbazol-9-yl)phenyl]-9-[4-(triphenylsilyl)phenyl]-9H-fluorenone, can also be used.

These electron blocking materials can be formed as a single film consisting of one type of the materials, or as a mixed film formed of plural types thereof. The electron blocking layer may be a monolayer structure of a single film or a mixed film, a laminate structure of multiple layers of a single film, a laminate structure formed of multiple layers of a mixed film, or also a laminate structure of one or more single layers and one or more mixed layers.

The absolute value of the HOMO energy level of the electron blocking material is preferably greater than the absolute value of the HOMO energy level of the hole transporting material (i.e., it has a deeper HOMO level). Specifically, the absolute value of the HOMO energy level of the electron blocking material is preferably 5.55 eV or more and 5.90 eV or less, more preferably 5.60 eV or more and 5.80 eV or less. The absolute value of the HOMO energy level of the electron blocking material is preferably as large as 0.05 eV or more and 0.45 eV or less of the absolute value of the HOMO energy level of the hole transporting material, more preferably as large as 0.05 eV or more and 0.35 eV or less, even more preferably as large as 0.10 eV or more and 0.35 eV or less.

### [Light Emitting Layer]

The light emitting layer is a layer that emits light after excitons are generated by the recombination of holes and electrons injected from the anode and cathode, respectively; and the light emitting layer may be used alone as a light emitting layer, but preferably contains a light emitting material and a host material.

The compound represented by the general formula (I) can be used as the host material. The compound represented by the general formula (I) has excellent hole transport property and has a wide band gap. Thereby, the organic EL device using the compound represented by the general formula (I) as the host material for the light emitting layer has a lower drive voltage and improved luminous efficiency. The compound represented by the general formula (I) used for the host material may be one or more of the compounds in the group of compounds represented by the general formula (I). The compound represented by the general formula (I) may be used in combination with other host materials.

Anthracene derivatives, heterocyclic compounds with an indole ring as a partial structure of the fused ring therein, heterocyclic compounds with a carbazole ring as a partial structure of the fused ring therein, carbazole derivatives, thiazole derivatives, benzimidazole derivatives, and polydialkylfluorene derivatives are also used as host materials. Examples of a hole injection and transport host material include carbazole derivatives such as 4,4'-di(N-carbazolyl)biphenyl (CBP), TCTA, and mCP, and examples of an electron transport host material include p-bis(triphenylsilyl)benzene (UGH2) and 2,2',2"-(1,3,5-phenylene)-tris(1-phenyl-1H-benzimidazole) (TPBI).

The light emitting material may be any of a fluorescent emitting material, phosphorescent emitting material, or delayed fluorescent material.

Examples of the light emitting material include metal complexes of quinolinol derivatives such as tris(8-quinolinolato)aluminum (Alq₃), various metal complexes, anthracene derivatives, bis-styrylbenzene derivatives, pyrene derivatives, oxazole derivatives, and poly(para-phenylenevinylene)derivatives, and further include quinacridone, coumarin, rubrene, perylene and derivatives thereof, benzopyran derivatives, rhodamine derivatives, and aminostyryl derivatives.

Metal complexes with iridium or platinum as the central metal can be used as phosphorescent emitting materials. For example, green phosphorescent emitting materials such as Ir(ppy)₃, blue phosphorescent emitting materials such as FIrpic and FIr6, and red phosphorescent emitting materials such as Btp₂Ir(acac) can be mentioned. The amount of the phosphorescent emitting material doped into the host material is preferably in the range of 1 to 30% by weight of the total light emitting layer to avoid concentration quenching.

Examples of the delayed fluorescent material include carbazolyl dicyanobenzene (CDCB) derivatives such as PIC-TRZ, CC2TA, PXZ-TRZ, and 4CzIPN. For specific examples of delayed fluorescent materials, reference can be made to Appl. Phys. Lett. 98, 083302 (2011).

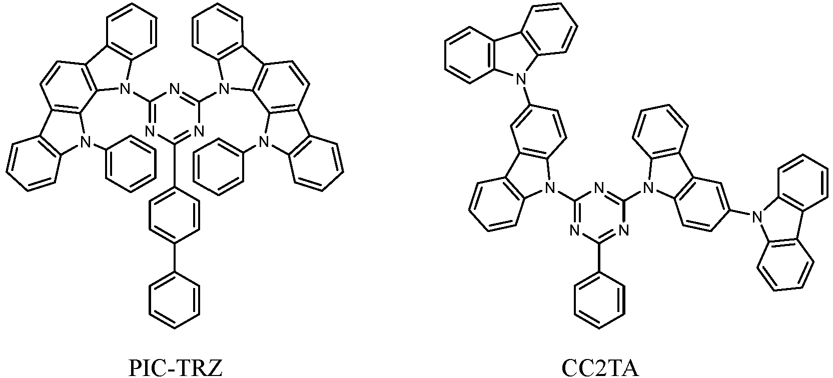

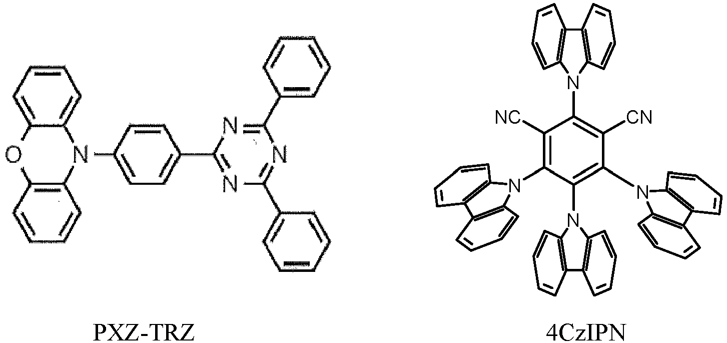

Compounds represented by the following general formula (III-1) or (III-2) can be preferably used as light emitting materials in the light emitting layer.

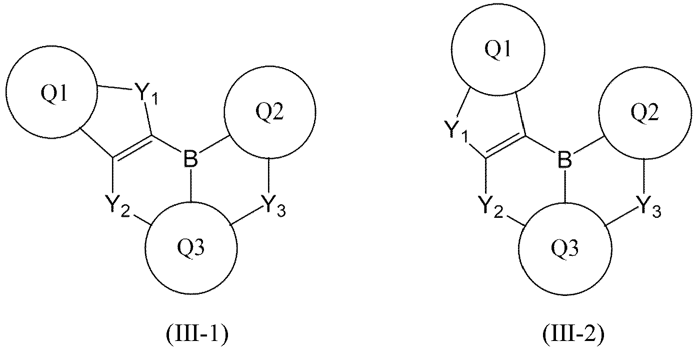

In the formulae, Q1 to Q3 may be identical or different from each other and represent a substituted or unsubstituted aromatic hydrocarbon ring with 6 to 50 carbon atoms, or a substituted or unsubstituted aromatic heterocyclic rings with 2 to 50 carbon atoms. Y₁ to Y₃ can be identical or different from each other and represent N-R₃, CR₄R₅, O, S, Se, or SiR₆R₇. R₃ to R₇ may be identical or different from each other and represent a hydrogen atom, a deuterium atom, a halogen atom, a nitro group, a cyano group, a substituted or unsubstituted alkyl group with 1 to 30 carbon atoms, a substituted or unsubstituted cycloalkyl group with 3 to 30 carbon atoms, a substituted or unsubstituted alkenyl group with 2 to 30 carbon atoms, a substituted or unsubstituted cycloalkenyl group with 3 to 30 carbon atoms, a substituted or unsubstituted heterocycloalkyl group with 1 to 30 carbon atoms, a substituted or unsubstituted monovalent aromatic hydrocarbon group with 6 to 50 carbon atoms, a substituted or unsubstituted monovalent aromatic heterocyclic group with 2 to 50 carbon atoms, a substituted or unsubstituted alkyloxy group with 1 to 30 carbon atoms, a substituted or unsubstituted aryloxy group with 6 to 30 carbon atoms, a substituted or unsubstituted alkylthio group with 1 to 30 carbon atoms, a substituted or unsubstituted arylthio group with 5 to 30 carbon atoms, a substituted or unsubstituted amino group with 0 to 30 carbon atoms, or a substituted or unsubstituted silyl group with 3 to 30 carbon atoms. R₃ to R₇ may each bond to any of Q1 to Q3 via a single bond, N, O, P or S, or fuse to form a fused ring, and R₄ and R₅, and R₆ and R₇ may each bond to each other to further form a ring. When Y₂ or Y₃ is N-R₃, at least one R₃ represents a group represented by the following general formula (IV-A) or the following general formula (IV-B).

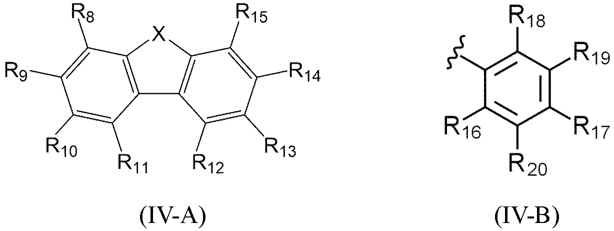

In the general formula (IV-A), X represents O or S. R₈ to R₁₅ may be identical or different from each other and represent a single bond, a hydrogen atom, a deuterium atom, a halogen atom, a hydroxy group, a nitro group, a cyano group, a substituted or unsubstituted alkyl group with 1 to 30 carbon atoms, a substituted or unsubstituted cycloalkyl group with 3 to 30 carbon atoms, a substituted or unsubstituted alkenyl group with 2 to 30 carbon atoms, a substituted or unsubstituted cycloalkenyl group with 3 to 30 carbon atoms, a substituted or unsubstituted alkynyl group with 2 to 30 carbon atoms, a substituted or unsubstituted heterocycloalkyl group with 1 to 30 carbon atoms, a substituted or unsubstituted aromatic hydrocarbon group with 6 to 50 carbon atoms, a substituted or unsubstituted aromatic heterocyclic group with 2 to 50 carbon atoms, a substituted or unsubstituted alkyloxy group with 1 to 30 carbon atoms, a substituted or unsubstituted aryloxy group with 6 to 30 carbon atoms, a substituted or unsubstituted alkylthio group with 1 to 30 carbon atoms, a substituted or unsubstituted arylthio group with 5 to 30 carbon atoms, a substituted or unsubstituted amino group with 0 to 30 carbon atoms, a substituted or unsubstituted silyl group with 3 to 30 carbon atoms, a substituted or unsubstituted germanium group with 0 to 30 carbon atoms, a substituted or unsubstituted boron group with 0 to 30 carbon atoms, a substituted or unsubstituted aluminum group with 0 to 30 carbon atoms, a substituted phosphoryl group with 0 to 30 carbon atoms, a substituted or unsubstituted seleno group with 0 to 30 carbon atoms, or a substituted or unsubstituted tellurium group with 0 to 30 carbon atoms. Any one of R₈ to R₁₅ bonds to N. R₈ to R₁₅ may bond to the adjacent group via a single bond, N, O, or S, or may fuse to form a ring.

In general formula (IV-B), R₁₆ represents a hydrogen atom, a deuterium atom, a substituted or unsubstituted alkyl group with 1 to 30 carbon atoms, a substituted or unsubstituted aromatic hydrocarbon group with 6 to 50 carbon atoms, or a substituted or unsubstituted aromatic heterocyclic group with 2 to 50 carbon atoms. R₁₇ represents a hydrogen atom, a deuterium atom, a substituted or unsubstituted alkyl group with 1 to 30 carbon atoms, a substituted or unsubstituted cycloalkyl group with 3 to 30 carbon atoms, a substituted or unsubstituted aromatic hydrocarbon group with 6 to 50 carbon atoms, or a substituted or unsubstituted aromatic heterocyclic group with 2 to 50 carbon atoms. R₁₈ to R₂₀ may be identical or different from each other and represent a hydrogen atom, a deuterium atom, a halogen atom, a cyano group, a substituted or unsubstituted alkyl group with 1 to 30 carbon atoms, a substituted or unsubstituted cycloalkyl group with 3 to 30 carbon atoms, a substituted or unsubstituted aromatic hydrocarbon group with 6 to 50 carbon atoms, a substituted or unsubstituted aromatic heterocyclic group with 2 to 50 carbon atoms, a substituted or unsubstituted alkyloxy group with 1 to 30 carbon atoms, a substituted or unsubstituted alkylthio group with 1 to 30 carbon atoms, a substituted or unsubstituted amino group with 0 to 30 carbon atoms, or a substituted or unsubstituted silyl group with 3 to 30 carbon atoms. The wavy line represents a bond with N. R₁₆ to R₂₀ may bond to the adjacent group via a single bond, N, O, or S, or may fuse to form a ring.

Q1 to Q3 in the general formulas (III-1) and (III-2) are preferably benzene rings. It is also preferable that the benzene rings of Q1 and Q2 have a benzofluoro or benzothieno fused ring structure. The substituent for the benzene ring of Q1 to Q3 is preferably a deuterium atom, an alkyl group with 1 to 30 carbon atoms, an aromatic hydrocarbon group with 6 to 50 carbon atoms, a diarylamino group with 12 to 30 carbon atoms, or a group formed by combining two or more these groups. Q2 in the general formulas (III-1) and (III-2) is also preferably a furan ring fused with a benzene ring (i.e., a benzofuro structure) or a thiol ring fused with a benzene ring (i.e., a benzothieno structure). Y₁ in the general formulas (III-1) and (III-2) is preferably O or S. Preferably, Y₂ and Y₃ each are independently N-R₃. R₃ is preferably a substituted or unsubstituted phenyl group (a ring may fuse to the phenyl group). The substituent is preferably a deuterium atom, an alkyl group with 1 to 30 carbon atoms, an aromatic hydrocarbon group with 6 to 50 carbon atoms, a dibenzofuryl group, a dibenzothienyl group, or a group formed by combining two or more these groups. The benzene ring to constitute the phenyl group can form a benzofluoro or benzothieno fused ring structure. Y₃ is also preferably O.

Preferred compounds of the general formulas (III-1) and (III-2) include a compound represented by the following general formula (III-3), a compound represented by the following general formula (III-4), a compound represented by the following general formula (III-5), and a compound represented by the following general formula (III-6).

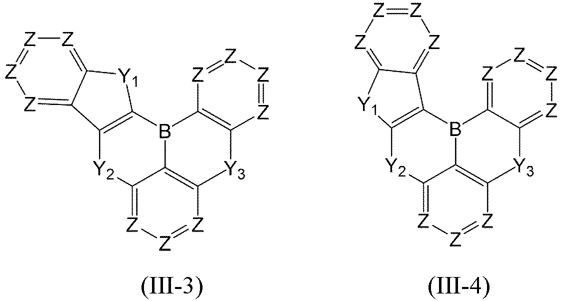

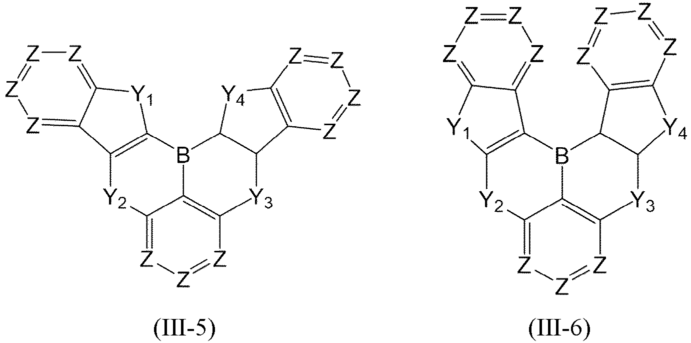

In the general formulas (III-3) to (III-6), Y₁ to Y₃ and R₃ to R₇ have the same meanings as in the general formulas (III-1) and (III-2) above. Y₄ represents N-R₃, C-R₄R₅, O, S, Se or Si-R₆R₇. Z may be identical or different from each other and represent N or CR₂₁. R₂₁ represent a hydrogen atom, a deuterium atom, a halogen atom, a cyano group, a substituted or unsubstituted alkyl group with 1 to 30 carbon atoms, a substituted or unsubstituted cycloalkyl group with 3 to 30 carbon atoms, a substituted or unsubstituted aromatic heterocyclic group with 2 to 50 carbon atoms, a substituted or unsubstituted alkyloxy group with 1 to 30 carbon atoms, a substituted or unsubstituted aryloxy group with 6 to 30 carbon atoms, a substituted or unsubstituted alkylthio group with 1 to 30 carbon atoms, a substituted or unsubstituted arylthio group with 5 to 30 carbon atoms, a substituted or unsubstituted alkylamino group with 1 to 30 carbon atoms, a substituted or unsubstituted arylamino group with 5 to 30 carbon atoms, a substituted or unsubstituted alkylsilyl group with 1 to 30 carbon atoms, or a substituted or unsubstituted arylsilyl group with 5 to 30 carbon atoms. R₂₁ may bond to the adjacent group via a single bond, N, O, or S, or may fuse to form a ring.

The definitions and details of the terms such as the groups in the general formulas (III-1) through (III-5) are the same as the definitions and descriptions of the terms of the corresponding groups described in the general formula (I).

The compound represented by the general formula (I) can be used in combination with the compound represented by the general formula (III-1) or (III-2) to achieve even better effects. Consequently, the invention can provide an electron blocking material comprising a compound represented by the general formula (I) for use in combination with a compound represented by the general formula (III-1) or (III-2); a laminate of a layer containing a compound represented by the general formula (III-1) or (III-2) and a layer containing a compound represented by the general formula (I) (preferably a laminate for luminescence); an organic EL device containing a compound represented by the general formula (III-1) or (III-2) and a compound represented by the general formula (I); an organic EL device having a layer containing a compound represented by the general formula (I) and a layer containing a compound represented by the general formula (III-1) or (III-2) (preferably, these two layers are adjacent to each other); and an organic EL device having an electron blocking layer containing a compound represented by the general formula (I) and a light emitting layer containing a compound represented by the general formula (III-1) or (III-2) (preferably, these two layers are adjacent to each other).

Furthermore, the compound can be used in combination with the compound represented by the general formula (II) to achieve excellent effects. Consequently, the invention can provide an electron blocking material comprising a compound represented by the general formula (I) for use in combination with both a compound represented by the general formula (II) and a compound represented by the general formula (III-1) or (III-2); a laminate of a layer containing a compound represented by the general formula (II), a layer containing a compound represented by the general formula (III-1) or (III-2), and a layer containing a compound represented by the general formula (I) (preferably a laminate for luminescence); an organic EL device containing a compound represented by the general formula (II), a compound represented by the general formula (III-1) or (III-2), and a compound represented by the general formula (I); an organic EL device comprising a layer containing a compound represented by the general formula (II), a layer containing a compound represented by the general formula (I), a layer containing a compound represented by the general formula (III-1) or (III-2) (preferably, these three layers are laminated sequentially adjacent to each other); an organic EL device comprising a hole transporting layer containing a compound represented by the general formula (II), an electron blocking layer containing a compound represented by the general formula (I), a light emitting layer containing a compound represented by the general formula (III-1) or (III-2) (preferably, these three layers are laminated sequentially adjacent to each other).

In the following, specific examples of the compounds represented by (III-1) or (III-2) are exemplified. However, the compounds represented by the general formula (III-1) or (III-2) employable in the invention should not be limitatively interpreted by these specific examples.

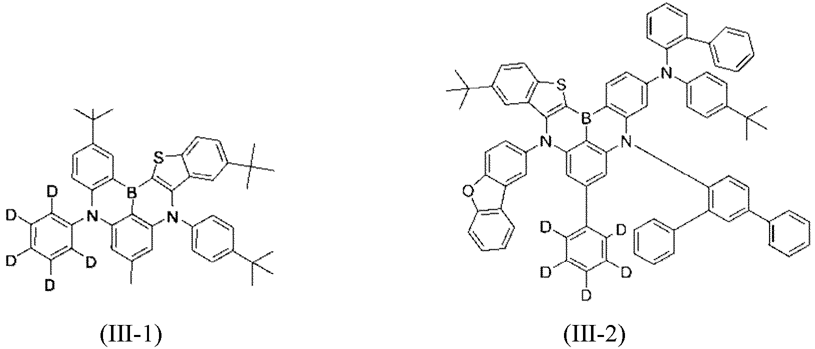

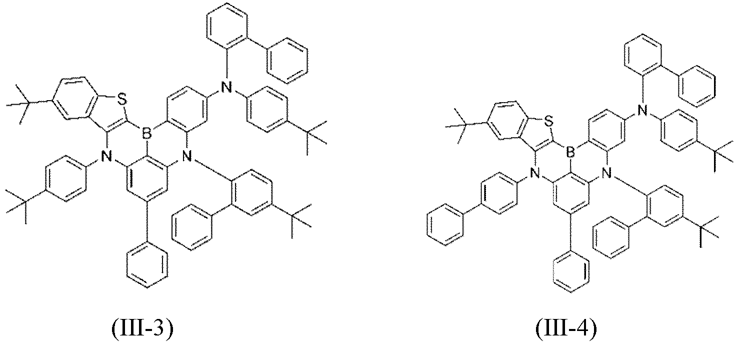

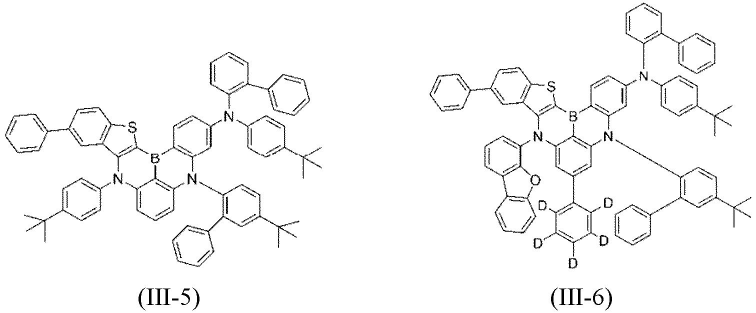

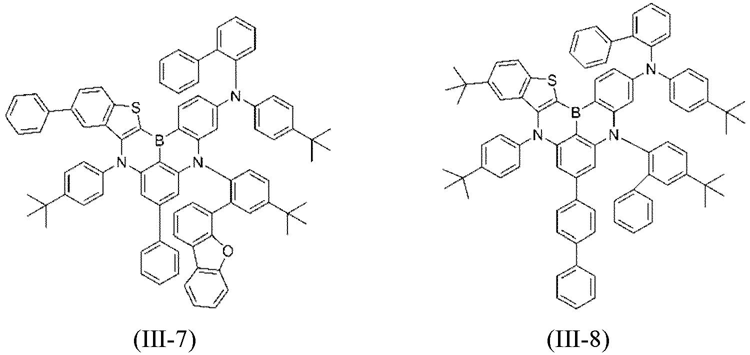

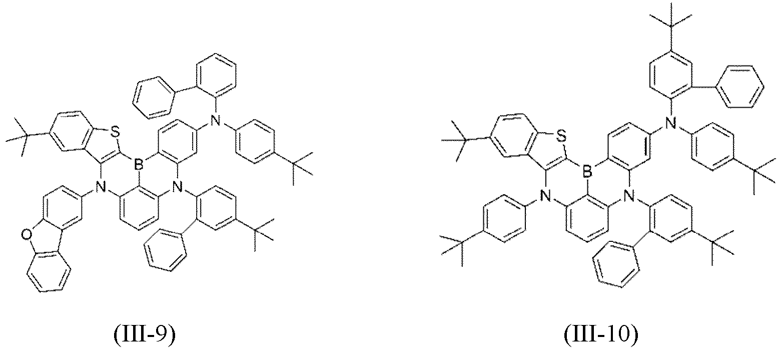

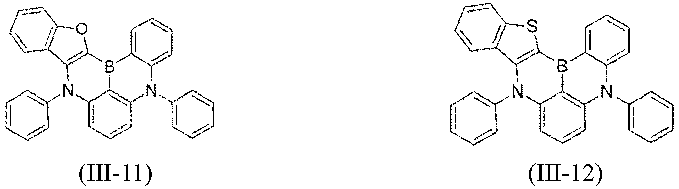

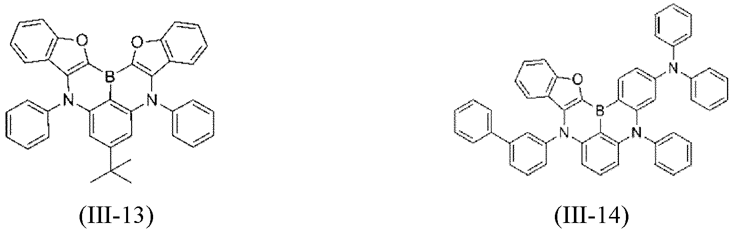

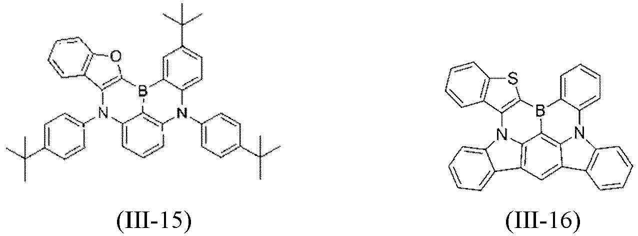

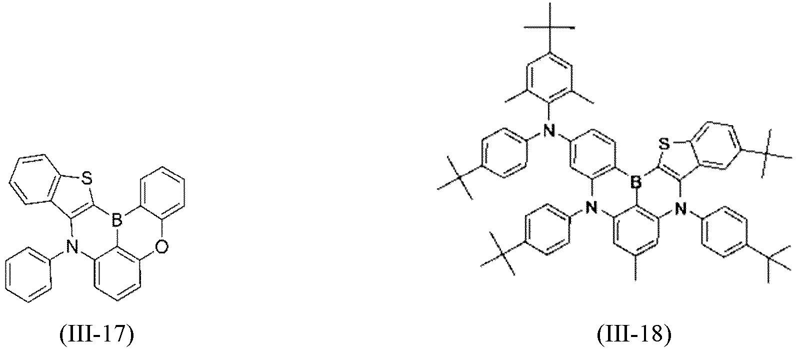

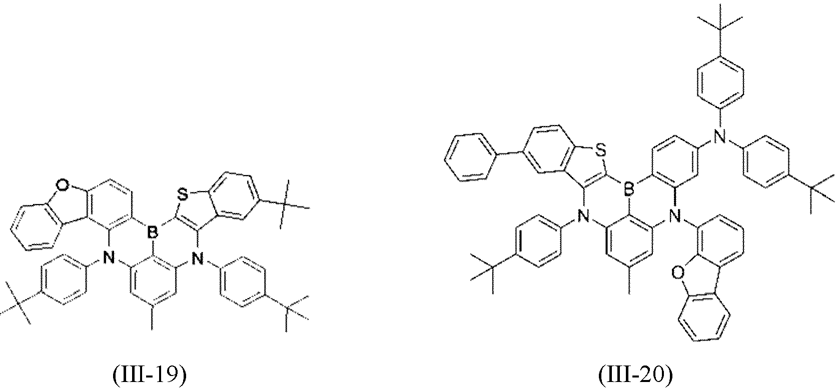

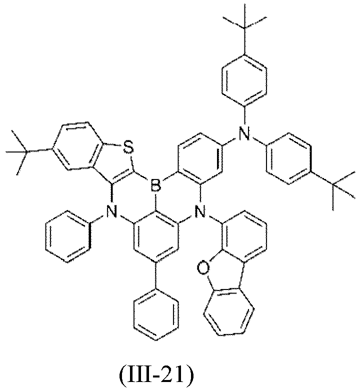

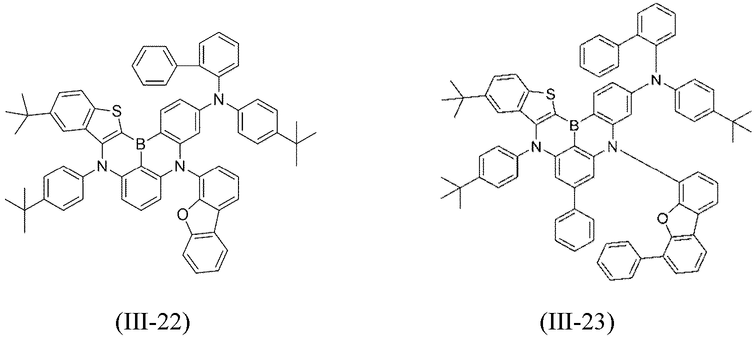

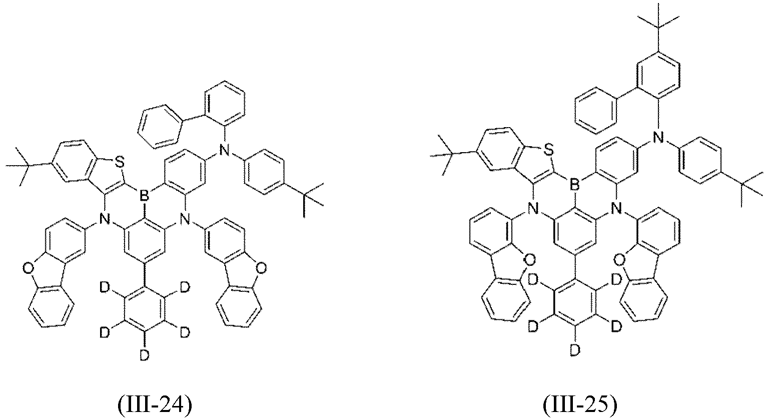

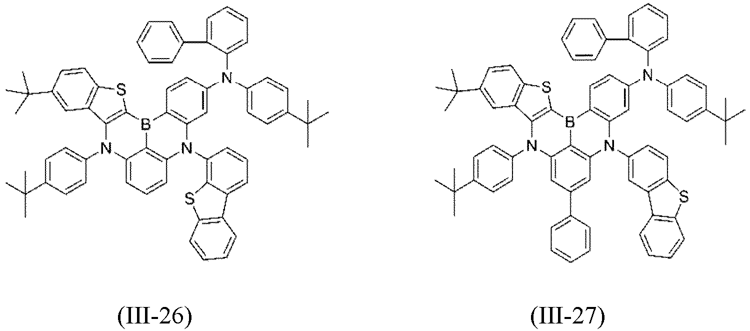

### [Hole Blocking Layer]

The hole blocking layer is placed, for example, between the light emitting layer and the electron transporting layer and is a layer having the function of inhibiting the diffusion of holes existing in the light emitting layer to the outside of the light emitting layer (on electron transporting layer side), thereby improving the recombination probability of electrons and holes in the light emitting layer. The hole blocking layer usually also has the function of transporting electrons. The hole blocking layer may also serve as an exciton blocking layer that suppresses the diffusion of excitons from the light emitting layer.

As materials for the hole blocking layer, compounds with hole-blocking effects, including phenanthroline derivatives such as bathocuproine (BCP), metal complexes of quinolinol derivatives such as bis(2-methyl-8-quinolinolate)-4-(phenylphenolato)aluminum (BAlq), various rare earth complexes, oxazole derivatives, triazole derivatives, and triazine derivatives, can be used. These materials may also serve as a role of electron transporting materials.

These materials can be formed as a single film consisting of one type of the material, or as a mixed film consisting of plural types thereof. The hole blocking layer may be a monolayer structure of a single film or a mixed film, a laminate structure of multiple layers of a single film, a laminate structure formed of multiple layers of a mixed film, or also a laminate structure of one or more single layers and one or more mixed layers.

### [Electron Transporting layer, Electron Injection Layer]

The electron injection layer is provided between the cathode and the light emitting layer or between the cathode and the electron transporting layer to lower the injection barrier of electrons supplied from the cathode, thereby lowering the drive voltage and improving the emission intensity.

The electron transporting layer is a layer that functions to transport electrons. The electron transporting layer may be an electron injection transport layer that also functions as an electron injection layer.

As materials for the electron injection layer, alkali metal salts such as lithium fluoride and cesium fluoride, alkaline earth metal salts such as magnesium fluoride, metal complexes of quinolinol derivatives such as lithium quinolinol, metal oxides such as aluminum oxide, as well as metals such as ytterbium (Yb), samarium (Sm), calcium (Ca), strontium (Sr), and cesium (Cs), can be used. The electron injection layer can be omitted in a preferred choice of an electron transporting layer and a cathode.

As materials for the electron transporting layer, metal complexes of quinolinol derivatives such as Alq₃ and BAlq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives and silole derivatives, can be used.

These materials can be formed as a single film consisting of one type of the material, or as a mixed film consisting of plural types thereof. The electron transporting layer may be a monolayer structure of a single film or a mixed film, a laminate structure of multiple layers of a single film, a laminate structure formed of multiple layers of a mixed film, or also a laminate structure of one or more single layers and one or more mixed layers.

The electron injection and hole transporting layers may also be composed of these electron injection or electron transporting materials with the addition of a metal such as cesium (N-type dopant).

### [Cathode]

For the cathode, employable herein are electrode materials of metals with a low work function, such as aluminum, as well as alloys with a lower work function, such as magnesium silver alloys, magnesium indium alloys, and aluminum magnesium alloys.

The layers constituting the organic EL device can be formed by known methods such as a vapor deposition method, a spin-coating method, or an ink-jet method.

### <Electronic Device>

The electronic device of the invention has a pair of electrodes and at least one organic layer arranged between the pair of electrodes, in which the at least one organic layer contains the compound represented by the general formula (I). For the description of the compound represented by the general formula (I), reference can be made to the description in the above section of <Compound represented by general formula (I)>.

Examples of the electronic device include displays and light-emitting devices equipped with an organic EL device, and additionally electrophotographic photoreceptors, image sensors, photoelectric conversion devices, and solar cells. Examples of the display device include display components such as organic EL panel modules, and also TVs, cell phones, tablets and personal computers. Examples of the light-emitting device include lighting devices or vehicle lights.

### [Example]

The characteristics of the invention will be described more specifically with reference to Synthesis Examples and Examples given below. The materials, processes and procedures shown below can be appropriately modified unless they deviate from the gist of the invention. Therefore, the scope of the invention is not limited to the following examples.

### [Example 1]

### <Synthesis of Compound (89)>

In a nitrogen-purged reaction vessel, N-phenyl-4-(phenylnaphthalen-3-yl)aniline: 10.0 g, 3-(4-chlorophenyl)-1-phenylnaphthalene: 9.3 g, sodium t-butoxide: 5.2 g, bis[tri(t-butylphosphine)]palladium(0): 0.3 g and toluene: 100 ml were put, and the mixture was stirred overnight under heat reflux. After confirming the completion of the reaction, the filtrate resulting from filtration was concentrated to give a crude product. The resultant crude product was purified by crystallization in a dichloromethane/acetone mixed solvent to give a compound (89): 14.4 g (yield: 82.3%).

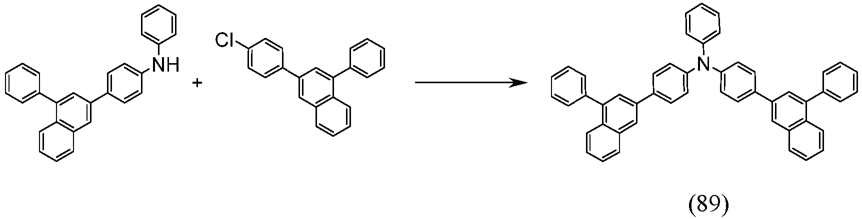

The structure of the resultant white powder was identified by NMR.

The following 35 hydrogen signals were detected in ¹H-NMR (CDCl₃).

δ(ppm)=8.05(2H), 7.95(2H), 7.90(2H), 7.71(2H), 7.67(4H), 7.65-7.49(10H), 7.47-7.40(4H), 7.31(2H), 7.26-7.21(6H), 7.08(1H).

### [Example 2]

### <Synthesis of Compound (13)>

In a nitrogen-purged reaction vessel, N-phenyl-4-(4-phenylnaphthalen-1-yl)aniline: 8.0 g, 1-(4-bromophenyl)-4-phenylnaphthalene: 8.5 g, tris(dibenzylideneacetone)palladium(0): 0.2 g, tri-tert-butylphosphine: 0.2 g, sodium tert-butoxide: 3.1 g and xylene: 80 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by crystallization in a toluene/acetone mixed solvent to give a compound (13): 4.3 g (yield: 31.0%).

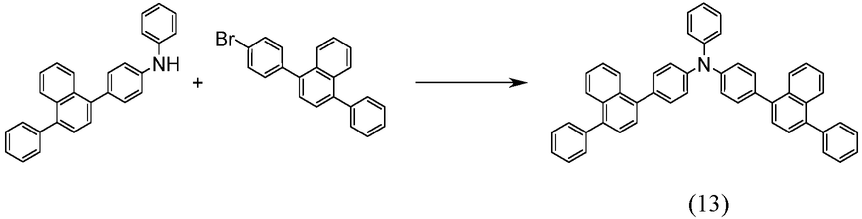

The structure of the resultant white powder was identified by NMR.

The following 35 hydrogen signals were detected in ¹H-NMR (CDCl₃).

δ(ppm)=8.15(2H), 8.00(2H), 7.59-7.46(22H), 7.43-7.35(8H), 7.15(1H).

### [Example 3]

### <Synthesis of Compound (23)>

In a nitrogen-purged reaction vessel, aniline: 1.8 g, 7-(4-chlorophenyl)-1-phenylnaphthalene: 12.2 g, tris(dibenzylideneacetone)palladium(0): 0.7 g, tri-tert-butylphosphine: 0.3 g, sodium tert-butoxide: 7.4 g and xylene: 180 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by recrystallization in an ethanol solvent to give a compound (23): 4.0 g (yield: 31.8%).

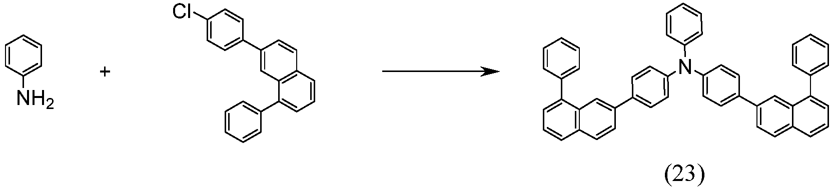

The structure of the resultant pale yellow powder was identified by NMR.

The following 35 hydrogen signals were detected in ¹H-NMR (CDCl₃).

δ(ppm)=8.12(2H), 7.99(2H), 7.90(2H), 7.78(2H), 7.59-7.51(14H), 7.48-7.44(4H), 7.30(2H), 7.20-7.16(6H), 7.08(1H).

### [Example 4]

### <Synthesis of Compound (106)>

In a nitrogen-purged reaction vessel, N-{4-(4-phenylnaphthalen-2-yl)phenyl}-[1,1'-biphenyl]-4-amine: 13.3 g, 3-(4-chlorophenyl)-1-phenylnaphthalene: 10.3 g, sodium t-butoxide: 5.7 g, bis[tri(t-butylphosphine)]palladium(0): 0.3 g and toluene: 130 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by recrystallization in an n-heptane solvent to give a compound (106): 18.4 g (yield: 85.5%).

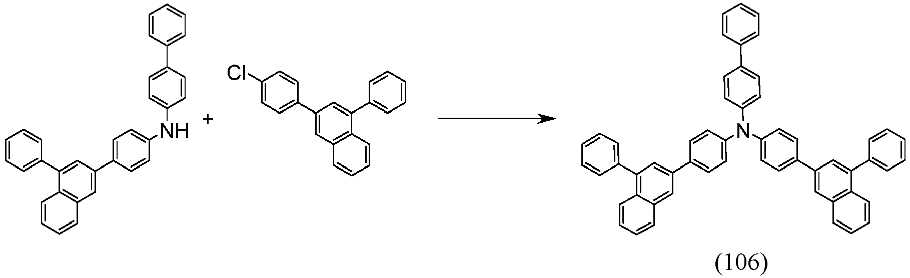

The structure of the resultant pale yellow powder was identified by NMR.

The following 39 hydrogen signals were detected in ¹H-NMR (CDCl₃).

δ(ppm)=8.09(2H), 7.99(2H), 7.93(2H), 7.75-7.71(6H), 7.64-7.43(21H), 7.36-7.29(6H).

### [Example 5]

### <Synthesis of Compound (115)>

In a nitrogen-purged reaction vessel, bis{4-(4-phenylnaphthalen-2-yl)phenyl}amine: 10.0 g, 1-bromobenzene-2,3,4,5,6-*d*₆: 3.4 g, tris(dibenzylideneacetone)palladium(0): 0.3 g, tri-tert-butylphosphine: 0.1 g, sodium tert-butoxide: 3.4 g and xylene: 100 ml were put, and the mixture was stirred overnight under heat reflux.

After confirming the completion of the reaction, the filtrate resulting from filtration was concentrated to give a crude product. The resultant crude product was purified by crystallization in a tetrahydrofuran/ethyl acetate mixed solvent to give a compound (115): 7.2 g (yield: 63.1%).

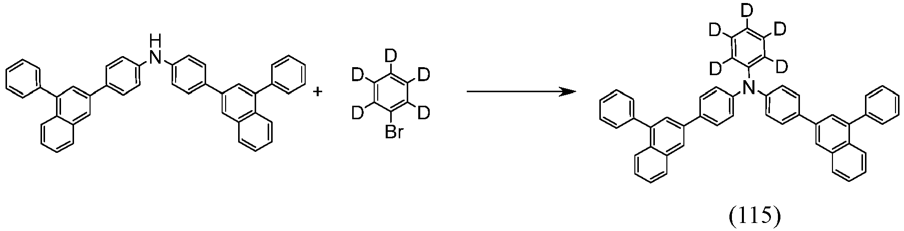

The structure of the resultant white powder was identified by NMR.

The following 30 hydrogen signals were detected in ¹H-NMR (CDCl₃).

δ(ppm)=8.88(2H), 7.97(2H), 7.92(2H), 7.74(2H), 7.70(4H), 7.60-7.52(10H), 7.50-7.42(4H), 7.27(4H).

### [Example 6]

### <Synthesis of Compound (116)>

In a nitrogen-purged reaction vessel, bis[4-{4-(phenyl-*d*₅)naphthalen-2-yl}phenyl]amine: 15.0 g, bromobenzene: 4.8 g, palladium(II) acetate: 0.1 g, tri-tert-butylphosphine: 0.5 g, sodium tert-butoxide: 3.7 g and toluene: 150 ml were put, and the mixture was stirred overnight under heat reflux.

After confirming the completion of the reaction, the filtrate resulting from filtration was concentrated to give a crude product. The resultant crude product was purified by crystallization in a dichloromethane/acetone mixed solvent to give a compound (116): 11.7 g (yield: 69.3%).

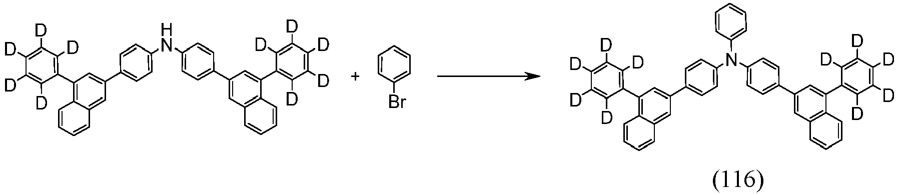

The structure of the resultant white powder was identified by NMR.

The following 25 hydrogen signals were detected in ¹H-NMR (CDCl₃).

δ(ppm)=8.07(2H), 7.99-7.91(4H), 7.74-7.69(6H), 7.54(2H), 7.44(2H), 7.36-7.10(9H).

### [Example 7]

### <Synthesis of Compound (117)>

In a nitrogen purged reaction vessel, aniline: 2.5 g, 3-(4-bromophenyl-2,3,5,6-*d*₄)-1-phenylnaphthalene: 21.5 g, tris(dibenzylideneacetone)palladium(0): 1.0 g, tri-tert-butylphosphine: 0.4 g, sodium tert-butoxide: 6.5 g, toluene: 150 ml were put, and the mixture was stirred overnight under heat reflux.

After confirming the completion of the reaction, the filtrate resulting from filtration was concentrated to give a crude product. The resultant crude product was purified by recrystallization in an acetone solvent to give a compound (117): 11.2 g (yield: 63.4%).

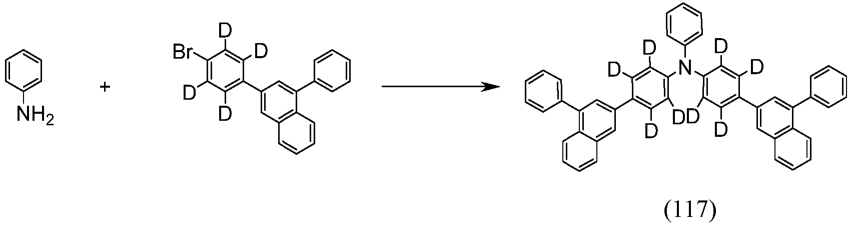

The structure of the resultant white powder was identified by NMR.

The following 27 hydrogen signals were detected in ¹H-NMR (CDCl₃).

δ(ppm)=8.08(2H), 7.98(2H), 7.92(2H), 7.74(2H), 7.60-7.52(10H), 7.50-7.42(4H), 7.34(2H), 7.26(2H), 7.11(1H).

### [Example 8]

### <Synthesis of Compound (118)>

In a nitrogen-purged reaction vessel, bis[4-{4-(phenyl-*d*₅)naphthalen-2-yl}phenyl]amine: 15.0 g, 1-bromobenzene-2,3,4,5,6-*d*₆: 5.0 g, palladium(II) acetate: 0.1 g, tri-tert-butylphosphine: 0.5 g, sodium tert-butoxide: 3.7 g and toluene: 150 ml were put, and the mixture was stirred overnight under heat reflux.

After confirming the completion of the reaction, the filtrate resulting from filtration was concentrated to give a crude product. The resultant crude product was purified by crystallization in a dichloromethane/acetone mixed solvent to give a compound (118): 12.8 g (yield: 75.1%).

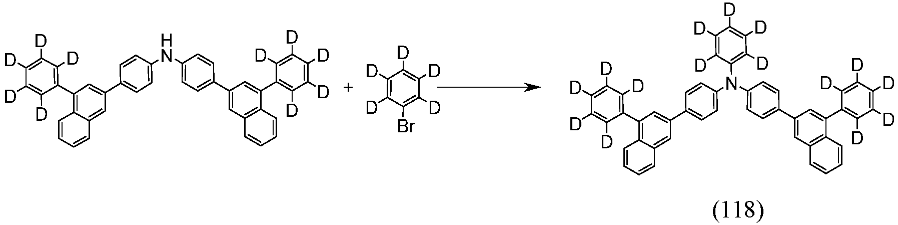

The structure of the resultant white powder was identified by NMR.

The following 20 hydrogen signals were detected in ¹H-NMR (CDCl₃).

δ(ppm)=8.07(2H), 7.99-7.91(4H), 7.74-7.69(6H), 7.53(2H), 7.44(2H), 7.28-7.10(4H).

### [Example 9]

### <Synthesis of Compound (171)>

In a nitrogen-purged reaction vessel, 4-{ 1-(dibenzofuran-4-yl)naphthalen-2-yl}-N-phenylaniline: 9.0 g, 7-(4-chlorophenyl)-1-phenylnaphthalene: 6.8 g, tris(dibenzylideneacetone)palladium(0): 0.2 g, tri-tert-butylphosphine: 0.2 g, sodium tert-butoxide: 2.8 g and toluene: 100 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by crystallization in a dichloromethane/methanol mixed solvent to give a compound (171): 11.5 g (yield: 80.0%).

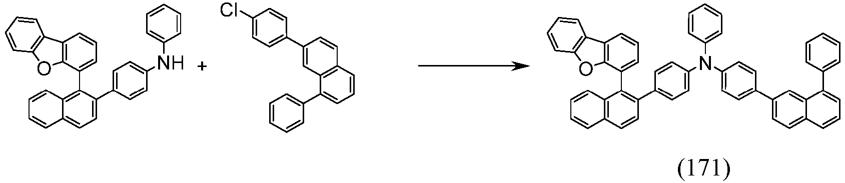

The structure of the resultant pale yellow powder was identified by NMR.

The following 37 hydrogen signals were detected in ¹H-NMR(DMSO-*d*₆).

δ(ppm)=8.17-8.07(5H), 7.99-7.94(2H), 7.80-7.72(2H), 7.62-7.27(17H), 7.17(2H), 7.04-6.97(3H), 6.72(2H), 6.64(2H), 6.53(2H).

### [Example 10]

### <Synthesis of Compound (174)>

In a nitrogen-purged reaction vessel, 4-{1-(dibenzofuran-4-yl)naphthalen-2-yl}-N-phenylaniline: 10.0 g, 4-{2-(4-chlorophenyl)naphthalen-1-yl}dibenzofuran: 9.6 g, tris(dibenzylideneacetone) palladium(0): 0.2 g, tri-tert-butylphosphine: 0.2 g, sodium tert-butoxide: 3.1 g and toluene: 100 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by recrystallization in a chlorobenzene solvent to give a compound (174): 10.0 g (yield: 55.5%).

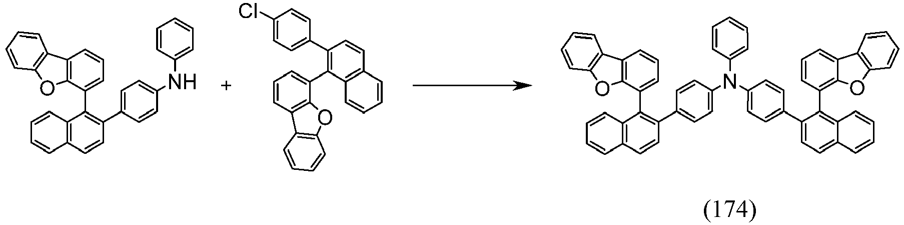

The structure of the resultant pale yellow powder was identified by NMR.

The following 39 hydrogen signals were detected in ¹H-NMR (DMSO-*d*₆).

δ(ppm)=8.17-8.06(8H), 7.69(2H), 7.58-7.56(4H), 7.49-7.31(12H), 7.06-7.00(2H), 6.91-6.84(5H), 6.35(2H), 6.24-6.18(4H).

### [Example 11]

### <Synthesis of Compound (177)>

In a nitrogen-purged reaction vessel, N-{4-(4-phenylnaphthalen-1-yl)phenyl}-[1,1'-biphenyl]-4-amine: 7.9 g, 1-(4-bromophenyl)-4-phenylnaphthalene: 7.0 g, tris(dibenzylideneacetone)palladium(0): 0.2 g, tri-tert-butylphosphine: 0.1 g, sodium tert-butoxide: 2.5 g and xylene: 80 ml were put, and the mixture was stirred overnight under heat reflux.

After confirming the completion of the reaction, the filtrate resulting from filtration was concentrated to give a crude product. The resultant crude product was purified by recrystallization in a toluene solvent to give a compound (177): 10.0 g (yield: 78.0%).

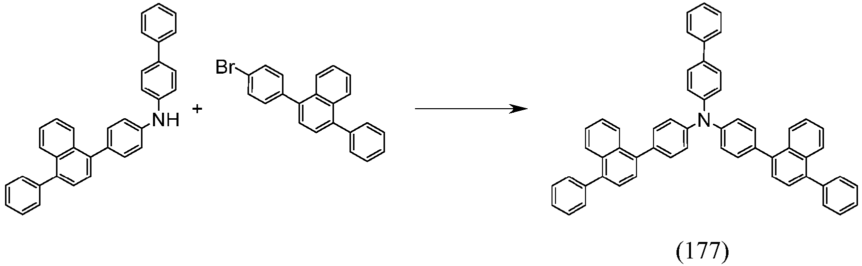

The structure of the resultant pale yellow powder was identified by NMR.

The following 39 hydrogen signals were detected in ¹H-NMR (CDCl₃).

δ(ppm)=8.17(2H), 8.02(2H), 7.68-7.63(4H), 7.60-7.47(24H), 7.42(6H), 7.37(1H).

### [Example 12]

### <Synthesis of Compound (183)>

In a nitrogen-purged reaction vessel, 4-([1,1'-binaphthalen]-3-yl)-N-phenylaniline: 10.0 g, 3-(4-chlorophenyl)-1-phenylnaphthalene: 7.8 g, tris(dibenzylideneacetone)palladium(0): 0.4 g, tri-tert-butylphosphine: 0.2 g, sodium tert-butoxide: 3.4 g and xylene: 100 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by recrystallization in an n-heptane solvent to give a compound (183): 12.6 g (yield: 75.9%).

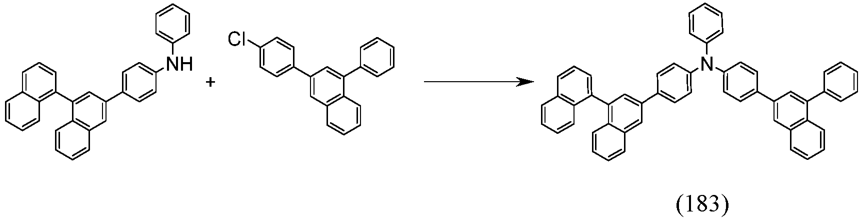

The structure of the resultant pale yellow powder was identified by NMR.

The following 37 hydrogen signals were detected in ¹H-NMR (CDCl₃).

δ(ppm)=8.18(1H), 8.07(1H), 8.07-7.96(4H), 7.93(1H), 7.83(1H), 7.74-7.68(5H), 7.64(1H), 7.51-7.41(12H), 7.36-7.23(10H), 7.10(1H).

### [Example 13]

### <Synthesis of Compound (184)>

In a nitrogen-purged reaction vessel, 4-([1,2'-binaphthalen]-3-yl)-N-phenylaniline: 15.6 g, 3-(4-chlorophenyl)-1-phenylnaphthalene: 11.7 g, tris(dibenzylideneacetone)palladium(0): 0.7 g, tri-tert-butylphosphine: 0.3 g, sodium tert-butoxide: 7.1 g and xylene: 160 ml were put, and the mixture was stirred overnight under heat reflux.

After confirming the completion of the reaction, the filtrate resulting from filtration was concentrated to give a crude product. The resultant crude product was purified by crystallization in a toluene/ethyl acetate mixed solvent to give a compound (184): 20.0 g (yield: 74.1%).

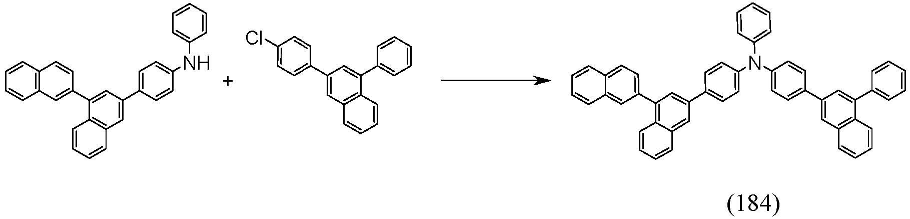

The structure of the resultant white powder was identified by NMR.

The following 37 hydrogen signals were detected in ¹H-NMR (CDCl₃).

δ(ppm)=8.12(1H), 8.07(2H), 8.01(2H), 7.99-7.92(5H), 7.84(1H), 7.74-7.69(6H), 7.60-7.52(8H), 7.50-7.43(3H), 7.34(2H), 7.30-7.24(6H), 7.11(1H).

### [Example 14]

### <Synthesis of Compound (186)>

In a nitrogen-purged reaction vessel, 4-([1,1'-binaphthalen]-3-yl)-N-phenylaniline: 7.5 g, 7-(4-chlorophenyl)-1-phenylnaphthalene: 6.2 g, bis[tri(t-butylphosphine)]palladium(0): 0.2 g, sodium tert-butoxide: 2.6 g and toluene: 70 ml were put, and the mixture was stirred overnight under heat reflux.

After confirming the completion of the reaction, the filtrate resulting from filtration was concentrated to give a crude product. The resultant crude product was purified by crystallization in a dichloromethane/acetone mixed solvent to give a compound (186): 10.0 g (yield: 80.0%).

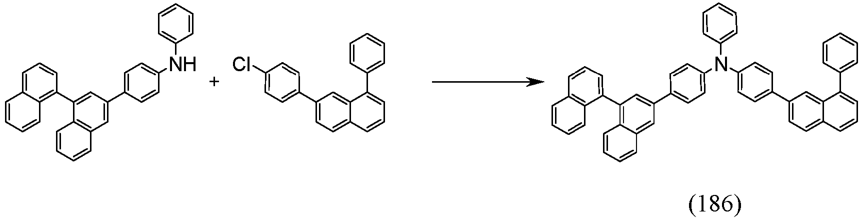

The structure of the resultant white powder was identified by NMR.

The following 37 hydrogen signals were detected in ¹H-NMR (CDCl₃).

δ(ppm)=8.16(1H), 8.11(1H), 8.01-7.97(4H), 7.89(1H), 7.81-7.76(2H), 7.09(2H), 7.63(1H), 7.58-7.41(15H), 7.34-7.27(3H), 7.24-7.18(6H), 7.08(1H).

### [Example 15]

### <Synthesis of Compound (190)>

In a nitrogen-purged reaction vessel, N-phenyl-4-(4-phenylnaphthalen-2-yl)-aniline: 7.0 g, 3-(3-chlorophenyl)-1,1'-binaphthalene: 7.6 g, bis[tri(t-butylphosphine)]palladium(0): 0.2 g, sodium tert-butoxide: 2.7 g and toluene: 70 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by recrystallization in an n-heptane solvent to give a compound (190): 11.9 g (yield: 90.2%).

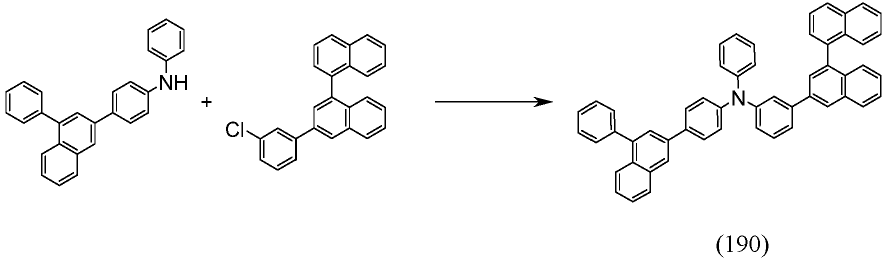

The structure of the resultant white powder was identified by NMR.

The following 37 hydrogen signals were detected in ¹H-NMR (CDCl₃).

δ(ppm)=8.10(1H), 8.06(1H), 8.00-7.95(4H), 7.92(1H), 7.76(1H), 7.72(1H), 7.68(2H), 7.63-7.38(16H), 7.34-7.23(8H), 7.17(1H), 7.08(1H).

### [Example 16]

### <Synthesis of Compound (191)>

In a nitrogen-purged reaction vessel, N-phenyl-4-(4-phenylnaphthalen-2-yl)-aniline: 10.0 g, 4-{2-(4-chlorophenyl)naphthalen-1-yl}dibenzofuran: 11.4 g, tris(dibenzylideneacetone)palladium(0): 0.5 g, tri-tert-butylphosphine: 0.2 g, sodium tert-butoxide: 3.1 g and xylene: 100 ml were put, and the mixture was stirred overnight under heat reflux.

After confirming the completion of the reaction, the filtrate resulting from filtration was concentrated to give a crude product. The resultant crude product was purified by crystallization in a toluene/acetone mixed solvent to give a compound (191): 14.7 g (yield: 73.8%).

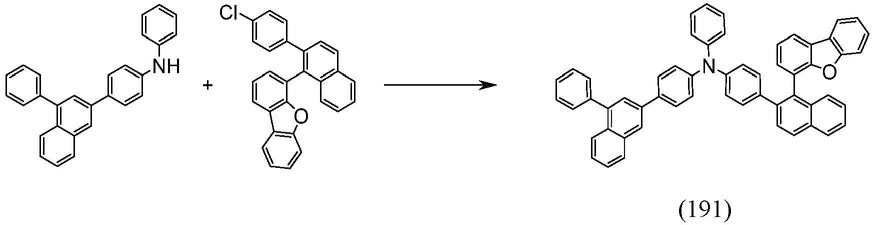

The structure of the resultant pale yellow powder was identified by NMR.

The following 37 hydrogen signals were detected in ¹H-NMR(DMSO-*d*₆).

δ(ppm)=8.21-8.16(4H), 8.09(2H), 7.80(1H), 7.75(1H), 7.67-7.56(10H), 7.53-7.37(8H), 7.19(2H), 7.06(2H), 7.00(1H), 6.74-6.69(6H).

### [Example 17]

### <Synthesis of Compound (192)>

In a nitrogen-purged reaction vessel, N-phenyl-4-(4-phenylnaphthalen-2-yl)-aniline: 8.0 g, 6-(4-chlorophenyl)-1-phenylnaphthalene: 7.5 g, bis[tri(t-butylphosphine)]palladium(0): 0.2 g, sodium tert-butoxide: 3.1 g and toluene: 100 ml were put, and the mixture was stirred overnight under heat reflux.

After confirming the completion of the reaction, the filtrate resulting from filtration was concentrated to give a crude product. The resultant crude product was purified by crystallization in a dichloromethane/acetone mixed solvent to give a compound (192): 10.3 g (yield: 73.6%).

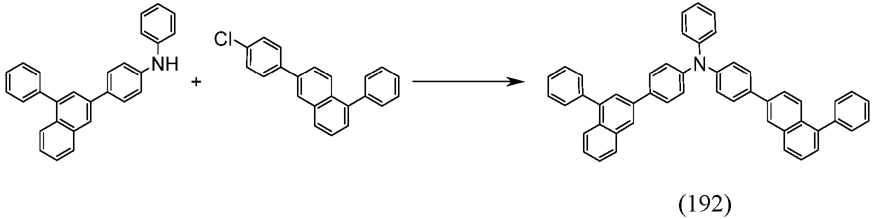

The structure of the resultant white powder was identified by NMR.

The following 35 hydrogen signals were detected in ¹H-NMR (CDCl₃).

δ(ppm)=8.13(1H), 8.07(1H), 7.99(2H), 7.92(2H), 7.79(1H), 7.73(1H), 7.69(2H), 7.60-7.43(16H), 7.33(2H), 7.26-7.20(6H), 7.10(1H).

### [Example 18]

### <Synthesis of Compound (195)>

In a nitrogen-purged reaction vessel, N-phenyl-4-{4-phenyl-*d*₅)naphthalen-2-yl}-aniline: 7.0 g, 7-(4-chlorophenyl)-1-phenylnaphthalene: 6.4 g, bis[tri(t-butylphosphine)]palladium(0): 0.2 g, sodium tert-butoxide: 2.7 g and toluene: 70 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by crystallization in a dichloromethane/acetone mixed solvent to give a compound (195): 8.1 g (yield: 66.5%).

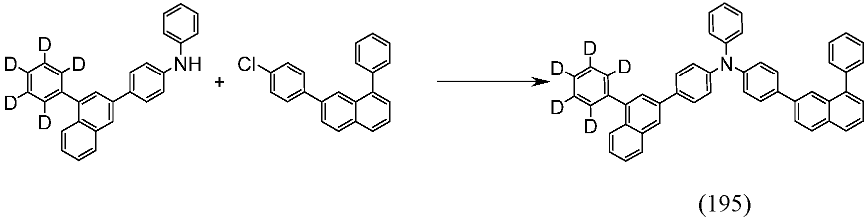

The structure of the resultant white powder was identified by NMR.

The following 30 hydrogen signals were detected in ¹H-NMR (CDCl₃).

δ(ppm)=8.14(1H), 8.07(1H), 7.99(2H), 7.94-7.89(2H), 7.79(1H), 7.74(1H), 7.69(2H), 7.60-7.52(8H), 7.48-7.43(3H), 7.33(2H), 7.26-7.20(6H), 7.10(1H).

### [Example 19]

### <Synthesis of Compound (198)>

In a nitrogen-purged reaction vessel, N-phenyl-4-(8-phenylnaphthalen-2-yl)aniline: 10.0 g, 2-(4-chlorophenyl)-6-phenylnaphthalene: 8.5 g, tris(dibenzylideneacetone)palladium(0): 0.5 g, tri-tert-butylphosphine: 0.2 g, sodium tert-butoxide: 3.1 g and xylene: 100 ml were put, and the mixture was stirred overnight under heat reflux.

After confirming the completion of the reaction, the filtrate resulting from filtration was concentrated to give a crude product. The resultant crude product was purified by recrystallization in a toluene solvent to give a compound (198): 10.9 g (yield: 62.3%).

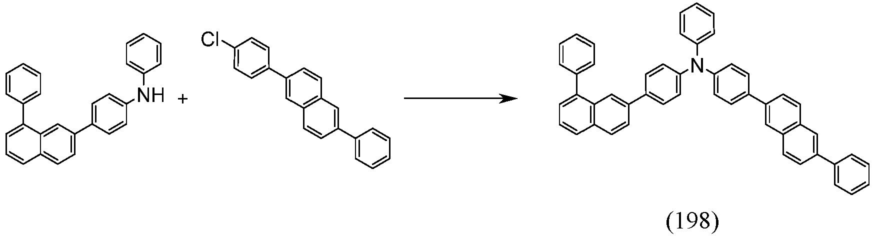

The structure of the resultant pale yellow powder was identified by NMR.

The following 35 hydrogen signals were detected in ¹H-NMR (DMSO-*d*₆).

δ(ppm)=8.24(2H), 8.11-8.05(3H), 8.01-7.98(2H), 7.98-7.79(7H), 7.61-7.35(14H), 7.17-7.13(7H).

### [Example 20]

### <Synthesis of Compound (199)>

In a nitrogen-purged reaction vessel, N-phenyl-4'-(8-phenylnaphthalen-2-yl)-[1,1'-biphenyl]-4-amine: 7.0 g, 7-(4-chlorophenyl)-1-phenylnaphthalene: 5.2 g, tris(dibenzylideneacetone)palladium(0): 0.1 g, tri-tert-butylphosphine: 0.1 g, sodium tert-butoxide: 2.3 g and toluene: 100 ml were put, and the mixture was stirred overnight under heat reflux.

After confirming the completion of the reaction, the filtrate resulting from filtration was concentrated to give a crude product. The resultant crude product was isolated by column chromatography (carrier: silica gel, eluent: dichloromethane/n-heptane) and purified by crystallization in a toluene/ethanol mixed solvent to give a compound (199): 8.6 g (yield: 76.0%).

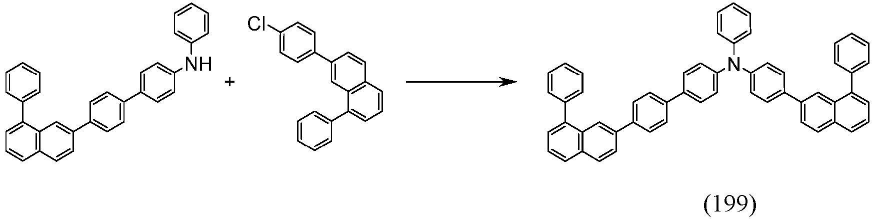

The structure of the resultant pale yellow powder was identified by NMR.

The following 39 hydrogen signals were detected in ¹H-NMR (DMSO-*d*₆).

δ(ppm)=8.15-8.07(3H), 8.00(3H), 7.93(1H), 7.86(1H), 7.76-7.45(22H), 7.36(2H), 7.13-7.09(7H).

### [Synthesis Example 1]

### <Synthesis of Q-1>

In a nitrogen-purged 100-mL reaction vessel, 1-bromobenzene (D-substituted): 16 mmol, 4-tert-butylaniline: 16 mmol, palladium(II) acetate: 0.1 g (1 mmol), sodium tert-butoxide: 3.0 g (32 mmol), bis(diphenylphosphino)-1,1'-binaphthyl: 0.2 g (1 mmol) and toluene: 45 mL were put, and the mixture was stirred under heat reflux for 24 hours. The reaction solution was cooled to room temperature and filtered under reduced pressure. The filtrate was purified by column chromatography to give a compound <Q-1>: 32.7 g (yield: 78.2%).

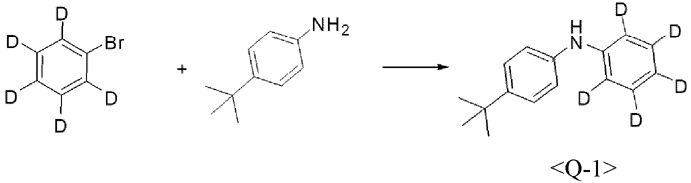

### <Synthesis of Q-2>

In a nitrogen-purged 250-mL reaction vessel, <P-5>: 98 mmol, <Q-1>: 98 mmol, palladium(II) acetate: 0.5 g (2 mmol), sodium tert-butoxide: 18.9 g (196 mmol), tri-tert-butylphosphine: 0.8 g (4 mmol) and toluene: 200 mL were put, and the mixture was stirred under heat reflux for 5 hours. The reaction solution was cooled to room temperature and filtered under reduced pressure. The filtrate was concentrated under reduced pressure, and purified by column chromatography to give a compound <Q-2>: 34.2 g (yield: 84.1%).

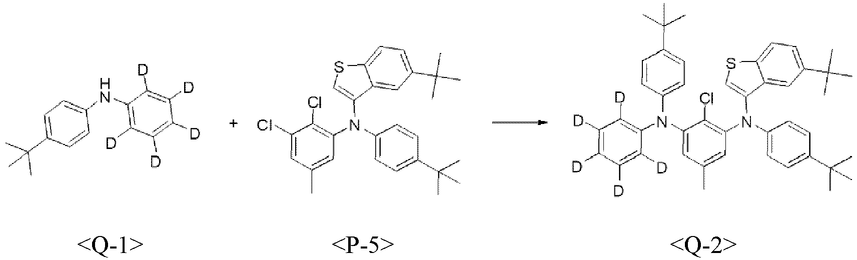

### <Synthesis of Compound (III-1)>

To a nitrogen-purged reaction vessel, <Q-2>: 23 mmol and tert-butylbenzene: 120 mL were added, then cooled to -78°C, and thereafter an n-hexane solution of 1.6 M n-butyllithium: 42.5 mL (68 mmol) was dropwise added thereto. The reaction solution was heated up to 60°C, stirred for 3 hours, and then nitrogen was blown to remove the hexane. The reaction solution was cooled to -78°C and boron tribromide: 11.3 g (45 mmol) was added dropwise. The mixture was stirred at room temperature for 1 hour, then N,N-diisopropylethylamine: 5.9 g (45 mmol) was added dropwise at 0°C, and stirred at 120°C for 2 hours. The reaction solution was allowed to cool to room temperature, an aqueous solution of sodium acetate was added, stirred for 1 hour, thereafter ethyl acetate was added, and the organic layer was separated. The organic layer was concentrated under reduced pressure, and purified by column chromatography to give a compound (III-1): 2.7 g (yield: 11.4%).

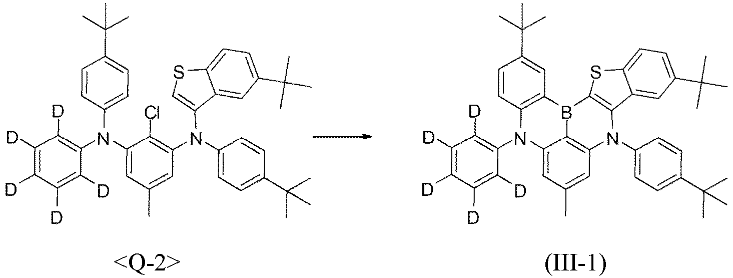

### [Synthesis Examples 2 to 27]

Compounds (III-2) through (III-27) were synthesized according to the same reaction as in Synthesis Example 1.

### [Example 21]

The melting point and the glass transition point of the compounds synthesized in Examples 1 to 20 were measured with a high-sensitivity differential scanning calorimeter (DSC 3100SA, manufactured by Bruker AXS). The results are shown in Table 1.

**Table 1**

| | Melting point (°C) | Glass transition point (°C) |
|---|---|---|
| Compound (89) | 216 | 110 |
| Compound (13) | - | 110 |
| Compound (23) | - | 110 |
| Compound (106) | - | 121 |
| Compound (115) | - | 109 |
| Compound (116) | - | 110 |
| Compound (117) | - | 110 |
| Compound (118) | - | 110 |
| Compound (171) | - | 130 |
| Compound (174) | 295 | 151 |
| Compound (177) | 240 | 123 |
| Compound (183) | - | 129 |
| Compound (184) | - | 121 |
| Compound (186) | - | 131 |
| Compound (190) | - | 121 |
| Compound (191) | - | 130 |
| Compound (192) | - | 110 |
| Compound (195) | - | 110 |
| Compound (198) | 216 | 105 |
| Compound (199) | - | 121 |

As shown in Table 1, the compounds synthesized in Examples 1 to 20 had a glass transition point above 100°C. This confirms that the compound represented by general formula (I) is stable in a thin film state.

### [Example 22]

Thin films of compounds synthesized in Examples 1 to 20 and compound (HTM-1) described below (e.g., PTL 8) were individually deposited on a substrate with ITO at a film thickness of 100 nm by vacuum evaporation. The HOMO energy level (HOMO level) of the thin film produced above was measured with an ionization potential measurement system (PYS-202, manufactured by Sumitomo Heavy Industries, Ltd.). Absolute values of the measured HOMO levels are shown in Table 2.

**Table 2**

| | HOMO level absolute value (eV) |
|---|---|
| Compound (89) | 5.63 |
| Compound (13) | 5.68 |
| Compound (23) | 5.63 |
| Compound (106) | 5.62 |
| Compound (115) | 5.66 |
| Compound (116) | 5.64 |
| Compound (117) | 5.66 |
| Compound (118) | 5.66 |
| Compound (171) | 5.67 |
| Compound (174) | 5.63 |
| Compound (177) | 5.67 |
| Compound (183) | 5.65 |
| Compound (184) | 5.64 |
| Compound (186) | 5.66 |
| Compound (190) | 5.79 |
| Compound (191) | 5.63 |
| Compound (192) | 5.64 |
| Compound (195) | 5.67 |
| Compound (198) | 5.64 |
| Compound (199) | 5.66 |
| HTM-1 | 5.57 |

As shown in Table 2, the compounds synthesized in Examples 1 to 20 have a larger absolute value of HOMO level than the absolute value of HOMO level (5.4 eV) of ordinary hole transporting materials such as NPD and TPD and than the compound (HTM-1), and had a deeper HOMO level. This confirms that the compound represented by the general formula (I) has good hole transport ability.

The above results confirm that the compound represented by the general formula (I) of the invention is useful as a host material for a hole transporting layer, electron blocking layer, or light emitting layer of an organic EL device.

### [Example 23]

The layer configuration of the organic EL device fabricated in this Example is shown in Fig. 1. In this Example, the organic EL device was fabricated by depositing a hole injection layer 3, a hole transporting layer 4, an electron blocking layer 5, a light emitting layer 6, an electron transporting layer 7, an electron injection layer 8, a cathode 9, and a capping layer 10 in this order on a glass substrate 1 on which a reflective ITO electrode has been pre-formed as a transparent anode 2.

Specifically, a glass substrate 1 having an ITO film with a thickness of 50 nm, a silver alloy reflective film with a thickness of 100 nm, and an ITO film with a thickness of 5 nm in this order was ultrasonically cleaned in isopropyl alcohol for 20 minutes and then dried on a hot plate heated at 250°C for 10 minutes. Then, after UV ozone treatment for 2 minutes, this glass substrate with a transparent anode was mounted in a vacuum evaporator and the pressure was reduced to 0.001 Pa or less.

Then, a hole injection layer 3 with a thickness of 10 nm was formed by binary evaporation of a compound (Acceptor-1) and a compound (HTM-1) to cover the transparent anode 2. The deposition rate ratio was Acceptor-1:HTM-1 = 3:97. On the hole injection layer 3, the compound (HTM-1) was vapor-deposited to form a hole transporting layer 4 with a thickness of 140 nm. On the hole transporting layer 4, a compound (89) was deposited to form an electron blocking layer 5 with a film thickness of 5 nm. On the electron blocking layer 5, a light emitting layer with a thickness of 20 nm was formed by binary evaporation of a compound (III-1, blue light emitting material) and a compound (EMH-1). The deposition rate ratio was III-1:EMH-1 = 5:95. On the light emitting layer 6, an electron transporting layer 7 with a thickness of 30 nm was formed by binary evaporation of a compound (ETM-1) and a compound (ETM-2). The deposition rate ratio was ETM-1:ETM-2 = 50:50. On the electron transporting layer 7, lithium fluoride was vapor-deposited to form an electron injection layer 8 with a thickness of 1 nm. On the electron injection layer 8, a cathode 9 was formed by depositing a magnesium silver alloy to a thickness of 12 nm. Finally, a compound (CPL-1) was deposited to form a capping layer 10 with a film thickness of 60 nm. Organic EL devices were fabricated according to the above process.

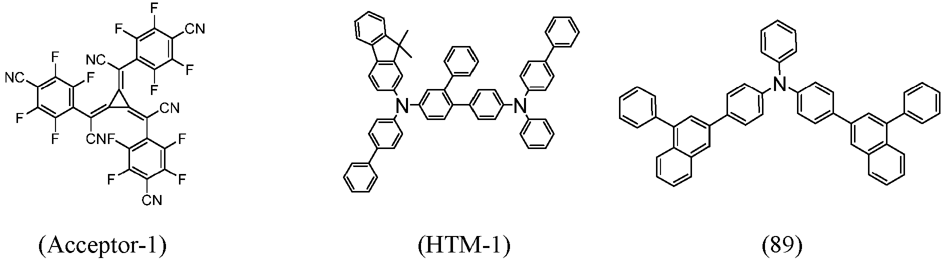

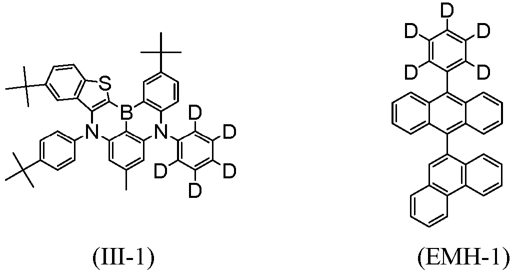

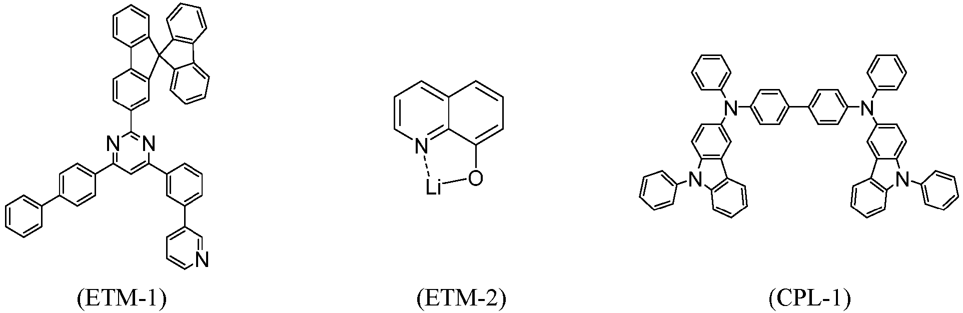

### [Examples 24 to 42]

Organic EL devices were fabricated under the same conditions as in Example 23, except that the compound shown in Table 3 below was used instead of the compound (89) as the material for the electron blocking layer 5.

### [Comparative Example 1]

For comparison, an organic EL device was fabricated under the same conditions as in Example 23, except that the following compound (HTM-2) was used instead of the compound (89) as the material for the electron blocking layer 5. 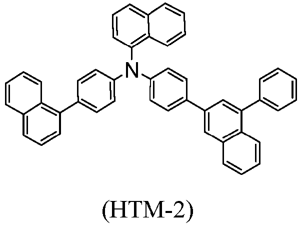 Compound described in PTL 7

### [Comparative example 2]

For comparison, an organic EL device was fabricated under the same conditions as in Example 23, except that the following compound (HTM-3) was used instead of the compound (89) as the material for the electron blocking layer 5. 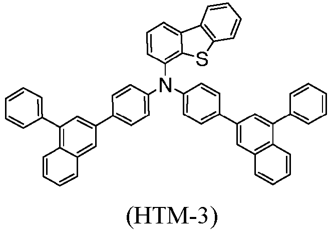 Compound described in PTL 7

### [Evaluation of EL Device]

DC voltage was applied to the organic EL device devices fabricated in Examples 23 to 42 and Comparative Examples 1 and 2 in air at room temperature, and the device characteristics and device lifetime were measured. The results are summarized in Table 3. The device lifetime was measured as the time required for the luminance to decay to 1900 cd/m² (equivalent to 95% when the initial luminance is set to 100%: 95% decay) when constant-current drive was performed with the luminance at the start of emission (initial luminance) set at 2000 cd/m².

**Table 3**

| | Electron Blocking Layer | Voltage [V] (@10mA/cm²) | Luminance [cd/m²] (@10mA/cm²) | Luminous Efficiency [cd/A] (@10mA/cm²) | Power Efficiency [lm/W] (@10mA/cm²) | Device Lifetime 95% Decay |
|---|---|---|---|---|---|---|
| Example 23 | Compound (89) | 3.48 | 930 | 9.31 | 8.42 | 488 hrs |
| Example 24 | Compound (13) | 3.47 | 887 | 8.87 | 8.02 | 463 hrs |
| Example 25 | Compound (23) | 3.49 | 935 | 9.36 | 8.43 | 453 hrs |
| Example 26 | Compound (106) | 3.46 | 950 | 9.52 | 8.66 | 450 hrs |
| Example 27 | Compound (115) | 3.49 | 892 | 8.93 | 8.05 | 453 hrs |
| Example 28 | Compound (116) | 3.49 | 880 | 8.81 | 8.01 | 478 hrs |
| Example 29 | Compound (117) | 3.46 | 938 | 9.39 | 8.54 | 451 hrs |
| Example 30 | Compound (118) | 3.46 | 935 | 9.33 | 8.37 | 457 hrs |
| Example 31 | Compound (171) | 3.50 | 910 | 9.09 | 8.16 | 457 hrs |
| Example 32 | Compound (174) | 3.50 | 905 | 9.05 | 8.11 | 473 hrs |
| Example 33 | Compound (177) | 3.50 | 930 | 9.30 | 8.36 | 460 hrs |
| Example 34 | Compound (183) | 3.50 | 910 | 9.12 | 8.20 | 487 hrs |
| Example 35 | Compound (184) | 3.50 | 925 | 9.27 | 8.32 | 477 hrs |
| Example 36 | Compound (186) | 3.48 | 922 | 9.21 | 8.32 | 465 hrs |
| Example 37 | Compound (190) | 3.48 | 907 | 9.06 | 8.17 | 459 hrs |
| Example 38 | Compound (191) | 3.51 | 929 | 9.27 | 8.36 | 457 hrs |
| Example 39 | Compound (192) | 3.49 | 910 | 9.09 | 8.19 | 469 hrs |
| Example 40 | Compound (195) | 3.49 | 920 | 9.21 | 8.32 | 486 hrs |
| Example 41 | Compound (198) | 3.51 | 930 | 9.30 | 8.35 | 485 hrs |
| Example 42 | Compound (199) | 3.49 | 925 | 9.25 | 8.36 | 454 hrs |
| Comparative Example 1 | HTM-2 | 3.60 | 822 | 8.24 | 7.19 | 364 hrs |
| Comparative Example 2 | HTM-3 | 3.57 | 826 | 8.26 | 7.28 | 349 hrs |

As shown in Table 3, the luminous efficiency when a current density of 10 mA/cm² was applied was 8.24 to 8.26 cd/A for the organic EL devices of Comparative Examples 1 to 2, while the organic EL devices of Examples 23 to 42 had a high efficiency of 8.81 to 9.52 cd/A. In terms of power efficiency, the organic EL devices of Examples 23 to 42 had a high efficiency of 8.01 to 8.66 lm/W, compared to 7.19 to 7.28 lm/W of the organic EL devices of Comparative Examples 1 to 2. Furthermore, in terms of device lifetime (95% decay), the organic EL devices of Examples 23 to 42 have a longer lifetime of 450 to 488 hours, compared to 349 to 364 hours for the organic EL devices of Comparative Examples 1 to 2.

### [Examples 43 to 120, Comparative Examples 3 to 5]

Organic EL devices were fabricated under the same conditions as in Example 23, except that the compound shown in Table 4 below was used instead of the compound (89) as the material for the electron blocking layer 5, and the compound shown in Table 4 below was used instead of (III-1) as the material for the light emitting layer 6. Table 4 below shows the results of evaluating the resultant organic EL devices, according to the same procedure as in the above [Evaluation of EL Device]. The data in Table 4 are shown as relative values to the results of the device in Comparative Example 3. The voltage is shown as the difference from the value of the device in Comparative Example 3, and the luminance, luminous efficiency, power efficiency, and device lifetime are shown as relative ratios (multipliers) to the value of the device in Comparative Example 3.

**Table 4**

| | Second hole transporting layer | Light Emitting Layer | Voltage [V] (@10mA/cm²) | Luminance [cd/m²] (@10mA/cm²) | Luminous Efficiency [cd/A] (@10mA/cm²) | Power Efficiency [lm/W] (@10mA/cm²) | Device Lifetime 95% Decay |
|---|---|---|---|---|---|---|---|
| Example 43 | Compound (89) | Compound (III-2) | -0.16 | 1.09 | 1.09 | *1.15* | 1.29 |
| Example 44 | Compound (183) | Compound (III-2) | -0.17 | 1.09 | 1.09 | *1.15* | 1.28 |
| Example 45 | Compound (184) | Compound (III-2) | -0.16 | 1.10 | 1.10 | *1.15* | 1.35 |
| Example 46 | Compound (89) | Compound (III-3) | -0.17 | 1.10 | 1.10 | *1.15* | 1.29 |
| Example 47 | Compound (183) | Compound (III-3) | -0.16 | 1.07 | 1.07 | 1.13 | 1.48 |
| Example 48 | Compound (184) | Compound (III-3) | -0.16 | 1.08 | 1.08 | 1.13 | 1.43 |
| Example 49 | Compound (89) | Compound (III-4) | -0.17 | 1.12 | 1.12 | 1.18 | 1.35 |
| Example 50 | Compound (183) | Compound (III-4) | -0.17 | 1.10 | 1.10 | *1.15* | 1.49 |
| Example 51 | Compound (184) | Compound (III-4) | -0.16 | 1.10 | 1.10 | *1.15* | 1.36 |
| Example 52 | Compound (89) | Compound (III-5) | -0.16 | 1.13 | 1.13 | 1.18 | 1.54 |
| Example 53 | Compound (183) | Compound (III-5) | -0.16 | 1.08 | 1.08 | 1.13 | 1.53 |
| Example 54 | Compound (184) | Compound (III-5) | -0.16 | 1.09 | 1.09 | 1.14 | 1.48 |
| Example 55 | Compound (89) | Compound (III-6) | -0.19 | 1.14 | 1.14 | 1.20 | 1.43 |
| Example 56 | Compound (183) | Compound (III-6) | -0.18 | 1.11 | 1.11 | 1.18 | 1.50 |
| Example 57 | Compound (184) | Compound (III-6) | -0.19 | 1.13 | 1.13 | 1.19 | 1.35 |
| Example 58 | Compound (89) | Compound (III-7) | -0.18 | 1.15 | 1.15 | 1.22 | 1.42 |
| Example 59 | Compound (183) | Compound (III-7) | -0.18 | 1.12 | 1.12 | 1.18 | 1.35 |
| Example 60 | Compound (184) | Compound (III-7) | -0.19 | 1.12 | 1.12 | 1.19 | 1.39 |
| Example 61 | Compound (89) | Compound (III-8) | -0.17 | 1.12 | 1.12 | 1.18 | 1.33 |
| Example 62 | Compound (183) | Compound (III-8) | -0.18 | 1.12 | 1.12 | 1.18 | 1.44 |
| Example 63 | Compound (184) | Compound (III-8) | -0.17 | 1.10 | 1.10 | 1.16 | 1.42 |
| Example 64 | Compound (89) | Compound (III-9) | -0.17 | 1.10 | 1.10 | 1.16 | 1.37 |
| Example 65 | Compound (183) | Compound (III-9) | -0.17 | 1.13 | 1.13 | 1.18 | 1.39 |
| Example 66 | Compound (184) | Compound (III-9) | -0.17 | 1.12 | 1.12 | 1.18 | 1.29 |
| Example 67 | Compound (89) | Compound (III-10) | -0.17 | 1.10 | 1.10 | 1.16 | 1.35 |
| Example 68 | Compound (183) | Compound (III-10) | -0.17 | 1.10 | 1.10 | 1.16 | 1.31 |
| Example 69 | Compound (184) | Compound (III-10) | -0.16 | 1.15 | 1.15 | 1.20 | 1.49 |
| Example 70 | Compound (89) | Compound (III-11) | -0.16 | 1.13 | 1.13 | 1.18 | 1.33 |
| Example 71 | Compound (183) | Compound (III-11) | -0.16 | 1.07 | 1.07 | 1.13 | 1.33 |
| Example 72 | Compound (184) | Compound (III-11) | -0.18 | 1.08 | 1.08 | 1.14 | 1.28 |
| Example 73 | Compound (89) | Compound (III-12) | -0.18 | 1.08 | 1.08 | 1.14 | 1.54 |
| Example 74 | Compound (183) | Compound (III-12) | -0.19 | *1.15* | *1.15* | 1.22 | 1.43 |
| Example 75 | Compound (184) | Compound (III-12) | -0.17 | 1.13 | 1.13 | 1.19 | 1.40 |
| Example 76 | Compound (89) | Compound (III-13) | -0.17 | 1.07 | 1.07 | 1.12 | 1.53 |
| Example 77 | Compound (183) | Compound (III-13) | -0.16 | 1.08 | 1.08 | 1.13 | 1.46 |
| Example 78 | Compound (184) | Compound (III-13) | -0.16 | 1.08 | 1.08 | 1.13 | 1.37 |
| Example 79 | Compound (89) | Compound (III-14) | -0.19 | 1.06 | 1.06 | 1.12 | 1.30 |
| Example 80 | Compound (183) | Compound (III-14) | -0.17 | 1.12 | 1.12 | 1.18 | 1.33 |
| Example 81 | Compound (184) | Compound (III-14) | -0.16 | 1.11 | 1.11 | 1.17 | 1.33 |
| Example 82 | Compound (89) | Compound (III-15) | -0.18 | 1.10 | 1.10 | 1.16 | 1.54 |
| Example 83 | Compound (183) | Compound (III-15) | -0.16 | 1.11 | 1.11 | 1.17 | 1.35 |
| Example 84 | Compound (184) | Compound (III-15) | -0.18 | 1.11 | 1.11 | 1.17 | 1.40 |
| Example 85 | Compound (89) | Compound (III-16) | -0.19 | 1.08 | 1.08 | 1.14 | 1.51 |
| Example 86 | Compound (183) | Compound (III-16) | -0.17 | 1.11 | 1.11 | 1.17 | 1.51 |
| Example 87 | Compound (184) | Compound (III-16) | -0.17 | 1.10 | 1.10 | 1.16 | 1.39 |
| Example 88 | Compound (89) | Compound (III-17) | -0.17 | 1.08 | 1.08 | 1.14 | 1.47 |
| Example 89 | Compound (183) | Compound (III-17) | -0.17 | 1.07 | 1.07 | 1.13 | 1.41 |
| Example 90 | Compound (184) | Compound (III-17) | -0.16 | 1.12 | 1.12 | 1.17 | 1.48 |
| Example 91 | Compound (89) | Compound (III-18) | -0.16 | 1.09 | 1.09 | 1.14 | 1.44 |
| Example 92 | Compound (183) | Compound (III-18) | -0.16 | 1.10 | 1.10 | 1.15 | 1.40 |
| Example 93 | Compound (184) | Compound (III-18) | -0.16 | 1.07 | 1.07 | 1.13 | 1.31 |
| Example 94 | Compound (89) | Compound (III-19) | -0.17 | 1.06 | 1.06 | 1.12 | 1.32 |
| Example 95 | Compound (183) | Compound (III-19) | -0.16 | 1.10 | 1.10 | 1.16 | 1.37 |
| Example 96 | Compound (184) | Compound (III-19) | -0.17 | 1.14 | 1.14 | 1.20 | 1.39 |
| Example 97 | Compound (89) | Compound (III-20) | -0.18 | 1.14 | 1.14 | 1.20 | 1.42 |
| Example 98 | Compound (183) | Compound (III-20) | -0.16 | 1.13 | 1.13 | 1.18 | 1.51 |
| Example 99 | Compound (184) | Compound (III-20) | -0.18 | 1.13 | 1.13 | 1.19 | 1.36 |
| Example 100 | Compound (89) | Compound (III-21) | -0.17 | 1.10 | 1.10 | *1.15* | 1.42 |
| Example 101 | Compound (183) | Compound (III-21) | -0.17 | 1.10 | 1.10 | 1.16 | 1.38 |
| Example 102 | Compound (184) | Compound (III-21) | -0.16 | 1.11 | 1.11 | 1.17 | 1.37 |
| Example 103 | Compound (89) | Compound (III-22) | -0.18 | 1.11 | 1.11 | 1.17 | 1.51 |
| Example 104 | Compound (183) | Compound (III-22) | -0.17 | 1.09 | 1.09 | 1.14 | 1.35 |
| Example 105 | Compound (184) | Compound (III-22) | -0.16 | 1.09 | 1.09 | 1.14 | 1.46 |
| Example 106 | Compound (89) | Compound (III-23) | -0.16 | 1.13 | 1.13 | 1.18 | 1.37 |
| Example 107 | Compound (183) | Compound (III-23) | -0.17 | 1.13 | 1.13 | 1.19 | 1.38 |
| Example 108 | Compound (184) | Compound (III-23) | -0.16 | 1.13 | 1.13 | 1.19 | 1.44 |
| Example 109 | Compound (89) | Compound (III-24) | -0.17 | 1.13 | 1.13 | 1.19 | 1.48 |
| Example 110 | Compound (183) | Compound (III-24) | -0.16 | 1.10 | 1.10 | *1.15* | 1.37 |
| Example 111 | Compound (184) | Compound (III-24) | -0.19 | 1.06 | 1.06 | 1.13 | 1.28 |
| Example 112 | Compound (89) | Compound (III-25) | -0.16 | 1.11 | 1.11 | 1.16 | 1.34 |
| Example 113 | Compound (183) | Compound (III-25) | -0.17 | 1.11 | 1.11 | 1.16 | 1.32 |
| Example 114 | Compound (184) | Compound (III-25) | -0.17 | 1.08 | 1.08 | 1.14 | 1.53 |
| Example 115 | Compound (89) | Compound (III-26) | -0.17 | 1.13 | 1.13 | 1.19 | 1.42 |
| Example 116 | Compound (183) | Compound (III-26) | -0.18 | 1.13 | 1.13 | 1.19 | 1.40 |
| Example 117 | Compound (184) | Compound (III-26) | -0.17 | 1.10 | 1.10 | 1.16 | 1.54 |
| Example 118 | Compound (89) | Compound (III-27) | -0.16 | 1.11 | 1.11 | 1.16 | 1.42 |
| Example 119 | Compound (183) | Compound (III-27) | -0.17 | 1.13 | 1.13 | 1.19 | 1.47 |
| Example 120 | Compound (184) | Compound (III-27) | -0.16 | 1.11 | 1.11 | 1.16 | 1.42 |
| Comparative Example 3 | Compound (HTM-2) | Compound (III-2) | 0 | 1 | 1 | 1 | 1 |
| Comparative Example 4 | Compound (HTM-3) | Compound (III-2) | 0.02 | 0.99 | 0.99 | 0.99 | 1.03 |
| Comparative Example 5 | Compound (89) | Compound (EMD-1) | -0.08 | 0.90 | 0.90 | 0.92 | 1.17 |

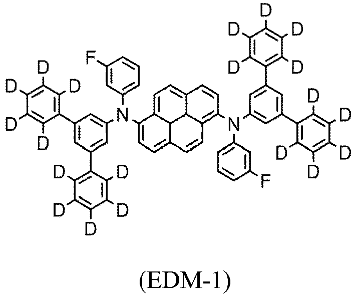

As obvious from the results in Tables 3 and 4, the compounds represented by the general formula (I) of the invention have good hole transport properties and excellent electron blocking ability compared to conventional hole transporting materials with an arylamine structure. Therefore, it has been found that the compounds represented by general formula (I) can be used to realize organic EL devices with high luminous efficiency and long lifetime compared to conventional organic EL devices. Such effects have also been found to be particularly more conspicuous when combined with the organoboron compound represented by the general formula (III-1) or (III-2).

### Industrial Applicability

The compound of the invention is excellent in electron blocking ability and hole transporting ability and has high thermal stability in a thin film state. Therefore, organic EL devices using the compound of the invention can achieve a low drive voltage, a high luminous efficiency, and a long device lifetime, and can be effectively used, for example, in displays and lighting devices for home appliances. Also, the compound of the invention can be applied in the field of electronic devices such as electrophotographic photoreceptors, image sensors, photoelectric conversion devices, and solar cells. Accordingly, the industrial applicability of the invention is great.

### Reference Signs List

1 : Glass Substrate
2: Transparent Anode
3: Hole Injection Layer
4: Hole Transporting Layer
5: Electron Blocking Layer
6: Light Emitting Layer
7: Electron Transporting Layer
8: Electron Injection Layer
9: Cathode
10: Capping Layer

## Claims

1. A compound represented by the following general formula (I): 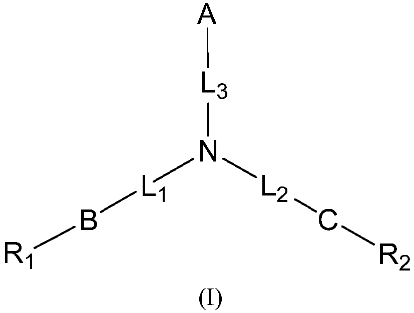 wherein,
A represents:
a substituted or unsubstituted monovalent aromatic hydrocarbon group, or
a substituted or unsubstituted monovalent aromatic heterocyclic group,
B and C can be the same as or different from each other,
each representing a heavy hydrogen-substituted or unsubstituted naphthylene group,
R₁ and R₂ can be the same as or different from each other,
each representing a heavy hydrogen-substituted or unsubstituted monovalent aromatic hydrocarbon group, or
a heavy hydrogen-substituted or unsubstituted monovalent aromatic heterocyclic group,
L₁, L₂ and L₃ can be the same as or different from each other,
each representing a single bond,
a heavy hydrogen-substituted or unsubstituted divalent aromatic hydrocarbon group, or
a heavy hydrogen-substituted or unsubstituted divalent aromatic heterocyclic group, provided that:
A-L₃ is not an unsubstituted aromatic heterocyclic group.

2. The compound according to claim 1, wherein, in the above general formula (I),
B and C can be the same as or different from each other,
each representing a heavy hydrogen-substituted or unsubstituted 1,2-naphthylene group,
a heavy hydrogen-substituted or unsubstituted 1,3-naphthylene group,
a heavy hydrogen-substituted or unsubstituted 1,4-naphthylene group,
a heavy hydrogen-substituted or unsubstituted 2,4-naphthylene group,
a heavy hydrogen-substituted or unsubstituted 2,5-naphthylene group,
a heavy hydrogen-substituted or unsubstituted 2,6-naphthylene group, or
a heavy hydrogen-substituted or unsubstituted 2,8-naphthylene group.

3. The compound according to claim 2, wherein, in the above general formula (I),
L₁ and L₂ each represent
a heavy hydrogen-substituted or unsubstituted phenylene group, or
a heavy hydrogen-substituted or unsubstituted biphenylylene group.

4. The compound according to claim 3, wherein, in the above general formula (I),
R₁ and R₂ can be the same as or different from each other,
each representing a heavy hydrogen-substituted or unsubstituted phenyl group,
a heavy hydrogen-substituted or unsubstituted naphthyl group,
a heavy hydrogen-substituted or unsubstituted dibenzofuranyl group,
a heavy hydrogen-substituted or unsubstituted phenanthrenyl group, or
a heavy hydrogen-substituted or unsubstituted biphenyl group.

5. The compound according to claim 4, wherein, in the above general formula (I),
A represents:
a substituted or unsubstituted phenyl group,
a substituted or unsubstituted naphthyl group,
a substituted or unsubstituted dibenzofuranyl group,
a substituted or unsubstituted dibenzothienyl group,
a substituted or unsubstituted phenanthrenyl group, or
a substituted or unsubstituted biphenyl group.

6. The compound according to claim 5, wherein, in the above general formula (I), B and C represent the same group.

7. The compound according to claim 6, wherein, in the above general formula (I), R₁ and R₂ represent the same group.

8. The compound according to claim 6, wherein, in the above general formula (I), R₁ and R₂ represent different groups.

9. The compound according to claim 5, wherein, in the above general formula (I), B and C represent different groups.

10. The compound according to claim 9, wherein, in the above general formula (I), R₁ and R₂ represent the same group.

11. The compound according to claim 9, wherein, in the above general formula (I), R₁ and R₂ represent different groups.

12. The compound according to claim 1, wherein an absolute value of the HOMO level of the compound represented by the above general formula (I), as measured with a thin film of 100 nm thickness prepared by vacuum evaporation of the compound represented by the above general formula (I) on a substrate with indium tin oxide, using an ionization potential measuring device in vacuum, is 5.60 eV or more and 5.80 eV or less.

13. An electron blocking material comprising the compound according to any one of claims 1 to 12.

14. An organic electroluminescent device having a pair of electrodes and organic layers containing at least a light emitting layer arranged between the pair of electrodes, wherein:
the organic electroluminescent device contains the compound according to any one of claims 1 to 12, in the at least one organic layer therein.

15. The organic electroluminescent device according to claim 14, wherein the at least one organic layer is an electron blocking layer and the electron blocking layer contains the compound.

16. The organic electroluminescent device according to claim 15, wherein the at least one organic layer is a hole transporting layer and the hole transporting layer contains a compound represented by the following general formula (II): 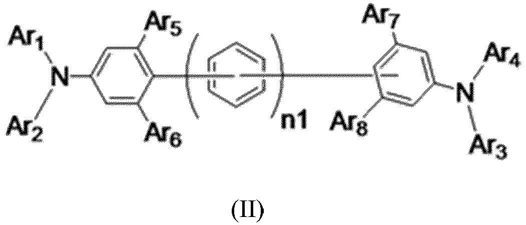
wherein Ar₁ to Ar₅ can be the same as or different from each other, representing a substituted or unsubstituted monovalent aromatic hydrocarbon group;
Ar₆ to Ar₈ can be the same as or different from each other, representing a hydrogen atom, or a substituted or unsubstituted monovalent aromatic hydrocarbon group, and at least two of Ar₆ to Ar₈ are hydrogen atoms;
n1 represents 0, 1 or 2;
Ar₃ and Ar₄ can bond to each other via a single bond to form a ring;
or can bond to each other via a substituted or unsubstituted methylene group, an oxygen atom, or a sulfur atom to form a ring.
Ar₃ or Ar₄ can bond to the benzene ring to which -N(Ar₃)(Ar₄) bonds via a single bond to form a ring, or can bond thereto via a substituted or unsubstituted methylene group, an oxygen atom, or a sulfur atom to form a ring.

17. The organic electroluminescent device according to claim 16, wherein an absolute value of the HOMO level of the compound represented by the above general formula (II), as measured with a thin film of 100 nm thickness prepared by vacuum evaporation of the compound represented by the above general formula (II) on a substrate with indium tin oxide, using an ionization potential measuring device in vacuum, is 5.50 eV or more and 5.65 eV or less.

18. The organic electroluminescent device according to claim 17, wherein an absolute value of the HOMO level difference between the compound represented by the above general formula (I) and the compound represented by the above general formula (II) is 0.05 eV or more and 0.35 eV or less.

19. An electronic device having a pair of electrodes and at least one organic layer arranged between the pair of electrodes, wherein the at least one organic layer contains the compound according to any one of claims 1 to 11.
